# EUROPEAN PATENT SPECIFICATION

(11) **EP 0 942 978 B1**
(45) Date of publication and mention of the grant of the patent: **28.08.2002**
(21) Application number: 97950165.7
(22) Date of filing: 11.11.1997
(51) Int. Cl.: C12N 15/12, C07K 14/47, C12Q 1/68, C12Q 1/00

(54) **METHODS FOR PRODUCING THE ANAPHASE PROMOTING COMPLEX**
VERFAHREN ZUR HERSTELLUNG VON DEM ANAPHASE-FÖRDERNDEN KOMPLEX
PROCEDES DE PRODUCTION D'UN COMPLEXE STIMULANT L'ANAPHASE

(30) Priority: 14.11.1996 EP 96118297
(43) Date of publication of application: 22.09.1999
(73) Proprietor: Boehringer Ingelheim International GmbH, 55218 Ingelheim am Rhein (DE)
(72) Inventor: NASMYTH, Kim, A-1010 Wien (AT); ZACHARIAE, Wolfgang, A-1190 Wien (AT); GALOVA, Marta, A-1040 Wien (AT)
(74) Representative: Laudien, Dieter, Dr.
(86) International application number: EP9706268
(87) International publication number: WO98021326

(56) References cited:
- WO-A-91/08739
- KING, RANDALL W. ET AL: "A 20S complex containing CDC27 and CDC16 catalyzes the mitosis-specific conjugation of ubiquitin to cyclin B" CELL (CAMBRIDGE, MASS.) (1995), 81(2), 279-88 CODEN: CELLB5;ISSN: 0092-8674, 1995, XP002030310 cited in the application
- STUART TUGENDREICH ET AL.: "CDC27Hs colocalizes with CDC16Hs to the centrosome and mitotic spindle and is essential for the metaphase to anaphase transition" CELL, vol. 81, no. 2, 21 April 1995, pages 261-268, XP002030311 NA US cited in the application
- STEFAN IRNIGER ET AL.: "Genes involved in sister chromatid separation are needed for B-type cyclin proteolysis in budding yeast" CELL, vol. 81, no. 2, 21 April 1995, pages 269-277, XP002030312 NA US cited in the application
- J.R. LAMB ET AL.: "Cdc16p, Cdc23p and Cdc27p form a complex essential for mitosis" EMBO JOURNAL, vol. 13, no. 18, 15 September 1994, pages 4321-4328, XP002030313 EYNSHAM, OXFORD GB cited in the application
- STUART TUGENDREICH ET AL.: "Linking yesat genetics to mammalian genomes: Identification and mapping of the human homolog of CDC27 via the expressed sequence tag (EST) data base" PROCEEDINGS OF THE NATIONAL ACADEMY OF SCIENCES OF USA, vol. 90, no. 21, 1 November 1993, pages 10031-10035, XP002030314 WASHINGTON US cited in the application
- WOLFGANG ZACHARIAE ET AL.: "TPR proteins required for anaphase progression mediate ubiquitination of mitotic B-type cyclins in yeast" MOLECULAR BIOLOGY OF THE CELL, vol. 7, no. 5, May 1996, pages 791-801, XP000671428 cited in the application
- ZACHARIAE, WOLFGANG ET AL: "Identification of subunits of the anaphase - promoting complex of Saccharomyces cerevisiae" SCIENCE (WASHINGTON, D. C.) (1996), 274(5290), 1201-1204 CODEN: SCIEAS;ISSN: 0036-8075, 1996, XP002064238

## Description

The invention relates to the regulation of the cell cycle in eucaryotic cells.

The aim of most chemotherapeutic approaches against cancer is to kill rapidly proliferating cells while leaving non-proliferating, differentiated cells unaffected. Since the state of the components regulating the cell cycle is different between proliferating and quiescent cells, these components are potential targets for anti-cancer drugs.

The invention relates to the use of a recently identified key regulator of the cell cycle, the Anaphase Promoting Complex or cyclosome, as a target for chemotherapeutic drugs.

It has been shown that mitotic cyclin degradation is required for the final exit from mitosis (Murray et al., 1989; Surana et al., 1993) and is a prerequisite for S-phase in the subsequent cell cycle (Dahman et al., 1995). In extracts from *Xenopus* eggs, degradation of cyclin B depends on a particle called the Anaphase Promoting Complex (APC), which has been shown to contain at least eight different proteins. The APC and the cyclosome, a particle found in clam oocytes, function as cell cycle regulated ubiquitin-protein ligases that mediate destruction box-dependent ubiquitination (King et al., 1995; Sudakin et al., 1995) and thereby target cyclins for proteolysis by the proteasome (Ciechanover, 1994; Peters, 1994). In the yeast *Saccharomyces cerevisiae*, the isolation of mutants defective in cyclin degradation led to the identification of the tetratricopetide repeat (TPR) proteins Cdc16p, Cdc23p, and Cdc27p as subunits of the APC (King et al., 1995; Imiger et al, 1995; Zachariae and Nasmyth, 1996). These proteins are required for the onset of anaphase in various organisms (Hirano et al., 1988; O'Donnell et al., 1991; Samejima and Yanagida, 1994; Tugendreich et al., 1995; Lamb et al., 1994). Because cyclin proteolysis per se is not required for anaphase (Surana et al., 1993; Holloway et al., 1993), it has been suggested that the APC also targets for destruction proteins whose degradation is necessary for sister chromatid separation (Irniger et al., 1995; Funabiki et al., 1996).

There are two main reasons why the APC fulfils the requirements of a target for chemotherapeutic drugs:
1. The activity of the APC is essential for sister chromatid separation and function of the mitotic spindle during cell proliferation. Interfering with its function would prevent tumor cells from completing mitosis.
2. Most tumor cells have highly abnormal karyotypes. They undergo anaphase in the presence of chromosomal damage that would prevent activation of the APC in normal cells. Tumor cells might therefore be especially sensitive to drugs that interfere with APC function.

The identification of all the subunits of the APC is a prerequisite for the understanding of the function and regulation of this key regulator of the cell cycle. In addition, the identification of the APC subunits provides a basis for interfering with the function of APC.

In a first set of experiments (Examples 1 - 7) of the present invention, two further subunits of the APC of the budding yeast *Saccharomyces cerevisiae* have been identified. It was further established that the yeast APC is a 36S particle that contains at least seven different proteins including the previously identified TPR proteins Cdc16p, Cdc23p, and Cdc27p. The first newly identified subunit is the largest subunit of the yeast APC. The sequence of *APC1* corresponds to the open reading frame YNL172W on chromosome XIV which was determined during the sequencing of the entire genome of *Saccharomyces cerevisiae.* The *APC1* sequence is currently available in the *Saccharomyces* Genome Database. In the experiments of the present invention, *APC1* was shown to be an essential gene. The Apc1 protein shows similarity to BIMEp from *Aspergillus nidulans* (Engle et al., 1990) and to the murine *tsg24* gene product (Starborg et al., 1994). The largest subunit of the APC therefore appears to be conserved between fungi and animal cells.

The second newly identified subunit is encoded by the *CDC26* gene (Akari et al., 1992). In the experiments of the present invention *CDC26* was shown to be essential for APC activity at elevated temperatures.

In a further set of experiments (Examples 8 -12) of the present invention, at least 12 different subunits were detected in the purified particle from budding yeast including the previously identified proteins Apc1p/BIMEp, Cdc16p, Cdc23p, Cdc26p, and Cdc27p. A combination of peptide mass mapping and tandem mass spectrometric sequencing allowed identification of five additional subunits (*APC2*, *APC5*, *APC4*, *APC9 and APC11*) purified in nanogram amounts. Apc2p, Apc5p and the RING finger protein Apc23p were found to be conserved from yeast to humans. The Apc2 protein is homologous to the cullin protein Cdc53p, which is a subunit of the S-phase promoting complex, an ubiquitin-ligase activity required for the initiation of DNA replication.

Human homologs of the yeast Cdc16 and Cdc27 proteins have been identified (Tugendreich et al. 1993; Tugendreich et al., 1995). They were shown to be subunits of a large complex. It can therefore be assumed that human homologs also exist for the remaining, as yet unidentified, subunits of the yeast APC. This notion is supported by the identification of a murine homolog (the *tsg24* gene product) of the yeast Apc1 protein. Other yeast APC subunits (the Apc2, Apc5 and Apc11 protein) were also shown to have mammalian homologs. The findings of the present invention are therefore highly relevant for human cells and provide a basis for the identification of subunits of the human APC. The identification of the human subunits is a prerequisite for establishing screening methods designed to find drugs that interfere with the function of the human APC. These drugs have the potential to inhibit rapidly proliferation cells such as tumor cells.

An efficient way to find substances that affect the activity of the APC is an assay that is capable of screening large numbers of compounds. To set up a suitable screening system for identifying such compounds, a source which provides large amounts of APC with a highly reproducible activity is required. Recombinant human APC is considered as the best source for such an assay.

The present invention provides a method for identifying substances that inhibit rapidly proliferating cells by interfering with the cells' entry into the subsequent cell cycle, characterized by the following steps
a) identifying novel subunits of the Anaphase Promoting Complex (APC) by
   i) replacing in a cell selected from cells from the budding yeast *Saccharomyces cerevisiae* or from human cells one or more endogenous genes encoding a known APC subunit by epitope-tagged versions of said genes or transforming the cell with a vector containing the corresponding epitope-tagged cDNA(s),
   ii) growing cells obtained in i) and preparing a protein extract,
   iii) isolating the APC by contacting the protein extract obtained in ii) with an antibody directed against the epitope-tag,
   iv) isolating the antibody-bound protein(s) and purifying them,
   v) determining the sequence of the protein(s), and optionally
   vi) identifying, in the case that the proteins obtained are yeast proteins, the human subunit(s) by comparing the sequence(s) of the yeast protein(s) and/or the DNA sequence encoding those proteins with published human sequences;
b) producing recombinant human or *Saccharomyces cerevisiae* APC containing APC subunits identified in a) by
   i) expressing cDNAs encoding APC subunits
   ii) isolating and purifying the subunits and allowing them to assemble to form a functional APC;
c) incubating the recombinant APC obtained in b), in the presence of a ubiquitin activating enzyme (E1) a suitable ubiquitin conjugating enzyme (E2), ubiquitin, ATP and an APC substrate, with a test substance, and
d) determining the effect of the test substance on the APC's ability to ubiquitinate the substrate.

According to one embodiment, the APC subunits are first identified in yeast. The yeast subunits are identified by purification of the APC particle from yeast. One or more genes encoding a known subunits (e.g. CDC16) are replaced by an epitope-tagged version (e.g. CDC16-myc6) and the APC is isolated from a protein extract prepared from such a strain. The essential part of the purification procedure is an immuno-affinity purification using one or more antibodies directed against the epitope tag(s) (e.g. the monoclonal antibody 9E10 directed against the myc epitope). Isogenic strains lacking the epitope tag are used as negative controls. Bound protein is separated by gel electrophoresis and subunits are identified by protein micro-sequencing or mass-spectrometry. Once the yeast subunits have been identified, the human homologues can be found by comparison with published sequences, e.g. by searching data bases. This method of the invention takes advantage of the fact that the genome of the budding yeast *Saccharomyces cerevisiae* has been fully sequenced. Therefore amino acid sequences obtained from the isolated subunits allow for direct identification of the respective gene.

According to an alternative embodiment of the method of the invention, the human APC or its subunits, respectively, can be directly isolated from human cells by a method related to the one described above.

The human cell line is established to express one or more epitope-tagged known human APC subunits such as CDC16Hs or CDC27Hs (Tugendreich et al., 1993; Tugendreich et al., 1995). For transient expression, the cells are transformed with a suitable vector containing the cDNA sequences encoding the known subunit(s). The APC is purified from an extract prepared from such a cell line by immuno-affinity purification using one or more antibodies against the epitope tags. Amino acid sequences determined from the purified proteins are used to search data bases. Alternatively, oligonucleotides may be designed that are based on the determined amino acid sequences and used as probes to screen cDNA libraries.

There is no limitation as to the use of epitope sequences for tagging the proteins; suitable epitope tags are commercially available or may be prepared according to standard methods; an example for methods for the construction of yeast strains expressing epitope-tagged APC subunits is described in Example 4A. If more than one subunits are tagged, the same or, preferably, different epitopes may be used for tagging. The purification of the APC by immunoprecipition can be carried out by standard methods, an example with the anti-myc antibody from strains expressing myc-tagged APC-subunits is described in Example 4C. For preparative purposes APC may be isolated from larger, unlabeled cultures, and proteins may be detected on gels by staining with silver or Coomassie Blue rather than autoradiography.

In a related aspect, the present invention further provides methods for producing functional APC complexes assembled from recombinant APC subunits.

Once the APC subunits have been identified and cDNA clones have been isolated, active complexes are generated using recombinant subunits.

In order to obtain the recombinant subunits, the cDNAs encoding the yeast or the human subunits, respectively, can be expressed, together or separately, using established expression systems such as Baculovirus or the yeast Pichia pastoris, which are well known in the art (O'Reilly et al., 1992) and commercially available, e.g. the Pichia pastoris system by Invitrogen. Both of these expression systems offer the possibility to simultaneously express several subunits to generate active complexes.

Unless otherwise stated, the individual steps of the methods of the invention, e.g. epitope-tagging, transformation of yeast strains or human cells, purification procedures, immunoreactions, cloning and expression, may be carried out according to standard protocols that are described in laboratory manuals, e.g. by Ausubel, et al., 1994; Harlow and Lane, 1988; Guthrie and Fink, 1991.

To determine whether the obtained APC complexes are functional and/or whether all the subunits are required for the APC to be functional, the preparation obtained by assembling the recombinantly produced subunits is tested for its ability to ubiquitinate known APC substrates such as cyclin B. An example for a suitable assay is described by Zachariae and Nasmyth, 1996.

Once active complexes have been generated, they can be used to screen for substances that interfere with the activity of the APC.

In an embodiment of the invention, the subunits that are used to form a functional APC are derived from yeast APC.

In a preferred embodiment of the invention, the subunits that are used to form a functional APC are derived from human APC.

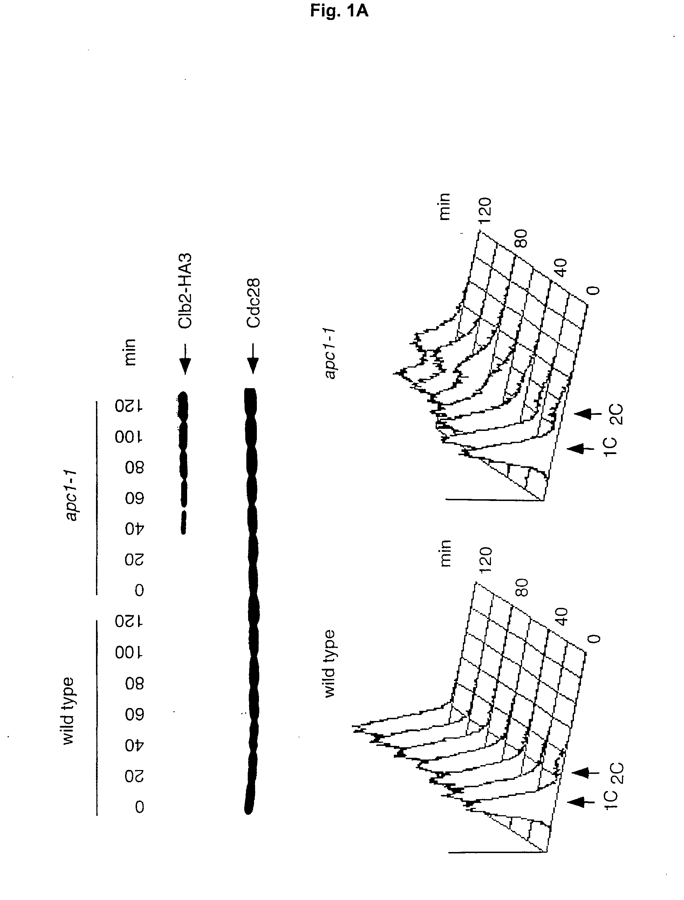

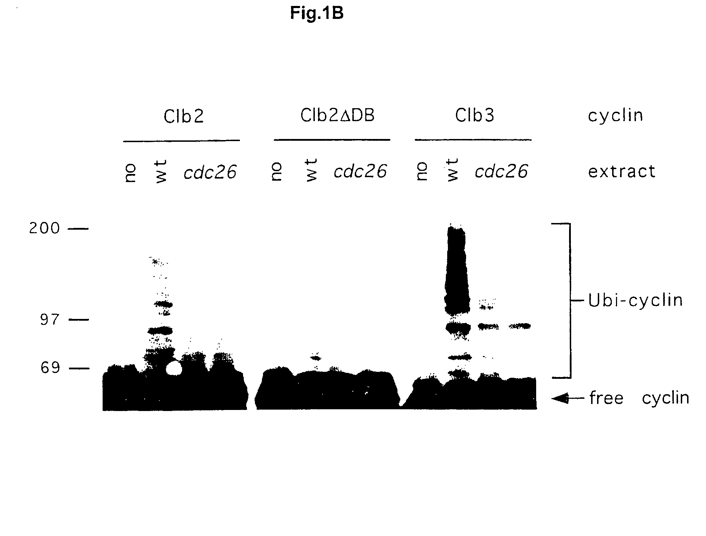

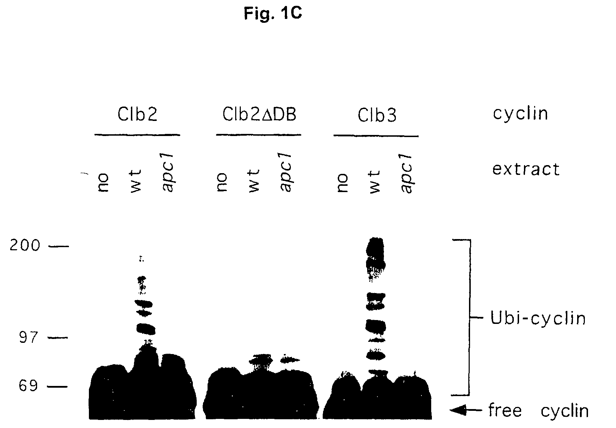

The screening methods of the invention are based on the principle to determine the ability of the APC to ubiquitinate a cyclin B containing substrate. This principle is described in the experiment shown in Fig. 1 of Zachariae and Nasmyth, 1996, in which yeast extracts that are capable of cell cycle regulated and destruction box-dependent ubiquitination were employed to show the ubiquitination of Clb2 in extracts from yeast strains overexpressing Clb2.

To perform such a screen, the recombinant APC is incubated in a reaction containing a ubiquitin activating enzyme (E1), a suitable ubiquitin conjugating enzyme (E2), ubiquitin, ATP and a substrate (e. g. cyclin B) which can be detected either by immunoblotting or because it is labeled radioactively. Cyclins containing a mutated or deleted destruction box can be used as negative control.

To convert this type of ubiquitination assay into a high throughput screening system, microtiter plates are coated with an APC substrate such as a cyclin B. The cyclins may be produced in recombinant form (see e.g. Glotzer et al., 1991); alternatively cell free extracts from cells overexpressing the respective cyclins may be used instead of the pure cyclins. The substrate is incubated with APC in the presence of ubiquitin-activating enzyme and ubiquitin conjugating enzyme (Rolfe-et al., 1995), ATP, an epitope-tagged ubiquitin (Ellison and Hochstrasser, 1991) and the test substance, and the amount of immobilized ubiquitin is measured using an enzyme (e. g. alkaline phosphatase or beta-galactosidase) linked to the anti-tag antibody which produces a color reaction. Substances that reduce the amount of immobilized ubiquitin are candidates for drugs that interfere with the cell cycle and thus inhibit the proliferation of rapidly proliferating cells, in particular tumor cells.

The yeast APC can be used in an initial screen for substances which interfere with the cell cycle in yeast. The ability of such substances to interfere with the ubiquitination of type B cyclins in higher eucaryotic cells, in particular in human cells, and therefore to inhibit rapidly proliferating cells, may then be confirmed in a secondary assay that employs the higher eucaryotic, in particular human, APC.

The present invention is based on the following findings and considerations:

Mitotic cyclins such as Clb2p are rapidly degraded in G1 arrested yeast cells (Irniger et al., 1995; Amon et al., 1994). To isolate mutants defective in cyclin proteolysis, in the experiments of the present invention mutagenized colonies were screened for β-galactosidase activity due to the accumulation of a Clb2-lacZ protein in G1 at 37°C as described (Irniger et al., 1995). 18 mutants were identified that arrested as large budded cells with a 2C DNA content after cycling cultures were shifted from 25 to 37°C. Complementation analysis and gene cloning demonstrated that new alleles of *CSE1* (Irniger et al., 1995), *CDC16* (King et al., 1995), *CDC26* (*cdc26-519*) and a mutant allele of a new gene have been isolated. The new gene was called *APC1*. *cse1* and *cdc16* mutants were previously identified in a similar screen (Irniger et al., 1995). The *CDC26* gene encodes an acidic protein of 17 kD (Akari et al., 1992).

Induction of a *GAL* promoter-*CLB2-HA3* fusion in G1 arrested cells at 35°C led to the accumulation of C1b2-HA3p within 20 min in the *apc1-1* mutant but not in wild-type cells. Mutant cells initiated S phase after 60 min but failed to bud whereas wild-type cells stayed arrested (Fig. 1A). A similar phenotype is caused by expression of a non-destructible version of Clb2p in wild-type cells (Amon et al., 1994). Extracts prepared from G1-arrested *cdc26-519* and *apc1-1* mutants were defective in ubiquitination of the mitotic cyclins Clb2p and Clb3p (Fig. 1, B and C), suggesting that reduced Clb2p proteolysis in the mutants stems from defective ubiquitination.

At 37°C *cdc26-519* mutants arrested as large-budded cells containing a short mitotic spindle in an undivided nucleus positioned at the bud-neck. Haploid cells lacking the entire *CDC26* coding sequence were viable at 25°C but arrested with a similar phenotype at 37°C, *CDC16-HA3 CDC26-myc9* cells grown at 25°C were shifted to 37°C and samples were taken for immunoblot analysis. Within two hours after the temperature shift the amount of Cdc26-myc9p increased 10-fold. The amount of Cdc16-HA3p did not increase.

The *APC1* wild-type gene encodes a 1748-amino acid (192-kD) protein whose COOH-terminal half is similar to the BIME protein from *A*. *nidulans* (Engle et al., 1990) and to a related protein encoded by the mouse *tsg24* gene (Starborg et al., 1994). An alignment of amino acids 904 to 1713 of Apc1p shows 28 % and 25 % identity to the corresponding regions of BIMEp (aa 1217 to 1939) and Tsg24p (aa 1026 to 1777), respectively. Tetrad analysis of spores derived from a diploid in which one copy of *APC1* was replaced by *HIS3* showed that *APC1* is an essential gene. His⁺ spores arrested as large budded cells after one or two cell divisions after germination.

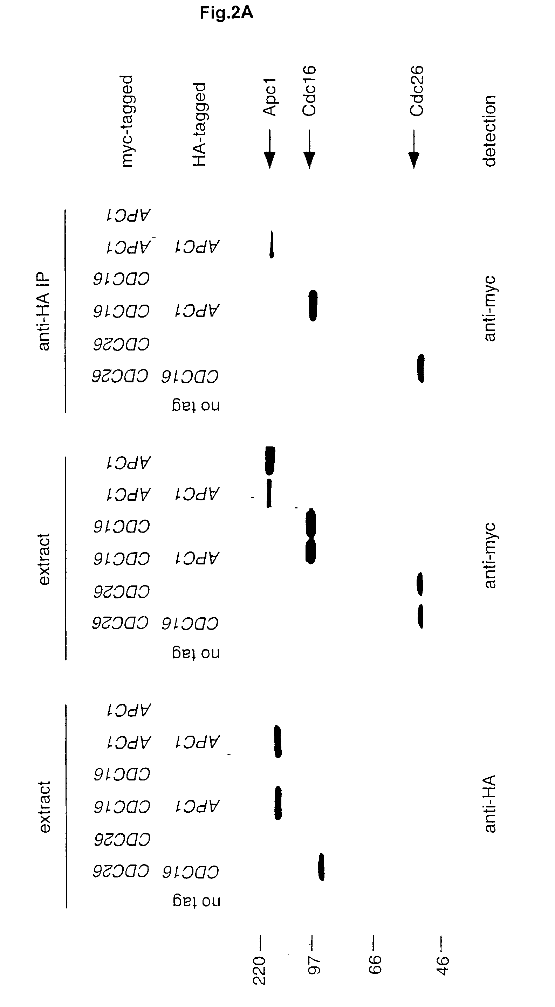

Cdc16p, Cdc23p, and Cdc27p associate with each other in yeast (Lamb et al., 1994). Cyclin proteolysis in vivo and cyclin ubiquitination in vitro also depends on the Cse1 protein (Irniger et al., 1995; Zachariae and Nasmyth, 1996). To investigate whether Cdc26p, Apc1p, and Cse1p associate with the Cdc16p-Cdc23p-Cdc27p complex, the endogenous genes were modified to encode variants carrying COOH-terminal HA (hemagglutinin) or myc epitope tags. All of these variants were fully functional. Extracts were prepared from strains expressing two proteins with different epitope tags and subjected to immunoprecipitations with the antibody to HA (Fig. 2A). Cdc16-myc6p was co-precipitated with Apc1-HA3p but not with Cse1-HA3p. Cdc26-myc9p was co-precipitated with Cdc16-HA3p. Apc1-myc6p was co-precipitated with Apc1-HA3p from an extract prepared from an *APC1-HA3*/*APC1-myc6* diploid strain. To characterize the complex further, various strains, each expressing a different myc-tagged protein, were labeled with ³⁵S methionine and cysteine. The antibody to the myc epitope precipitated the same set of proteins from extracts of *CDC16-myc6, CDC23-myc9, CDC26-myc9*, *CDC27-myc9* and *APC1-myc18* cells. This set included Cdc16p, Cdc23p, Cdc27p and Apc1p, which *were* identified by the increased molecular weight of epitope-tagged variants, and a protein of 80 kD. A protein with a size close to that of Cdc16p (100 kD) was detected in the immunoprecipitate from the *CDC16-myc6* strain. None of these proteins were co-precipitated with Cse1-myc9p. These data together with previous work (Zachariae and Nasmyth, 1996; Lamb et al., 1994) suggest that cyclin ubiquitination in yeast depends on a complex containing at least two molecule each of Apc1p, Cdc16p, Cdc23p, and Cdc27p. Cdc26p and at least two unidentified proteins (p80 and p100) are also components of this complex.

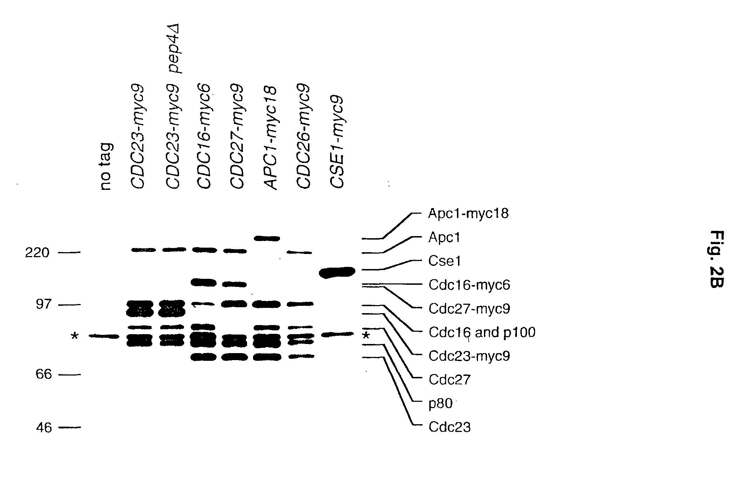

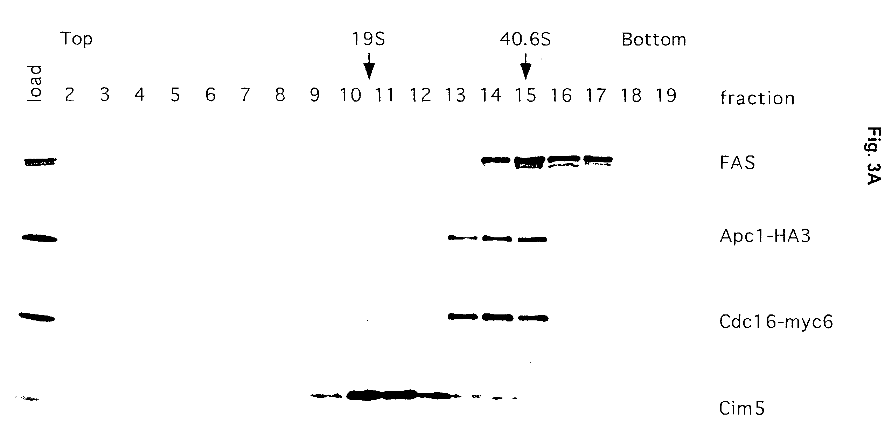

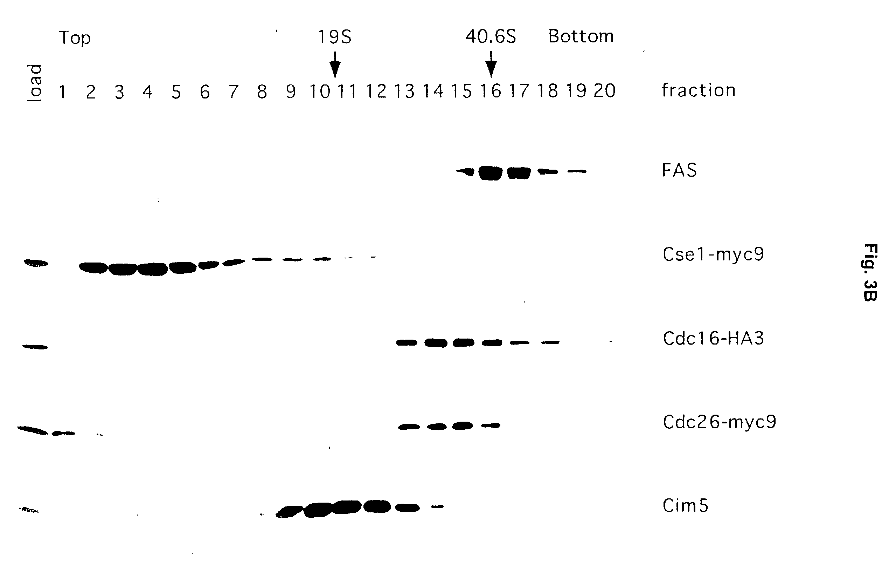

To determine the size of the yeast APC, extracts from strains expressing two epitope-tagged proteins were analyzed by glycerol density gradient centrifugation. Apc1-HA3p co-sedimented with Cdc16-myc6p as a 36S particle (Fig. 3A). Cdc23-HA3p co-sedimented with Cdc16-myc6p with the same velocity. The size of the yeast complex is larger than that reported for the *Xenopus* APC (20S) (King et al., 1995) and the cyclosome from clam (Sudakin et al., 1995). Cdc26-myc9p but not Cse1-myc9p co-sedimented with Cdc16-HA3p (Fig. 3B). These results and the immunoprecipitation data indicate that Cselp is not a component of the APC.

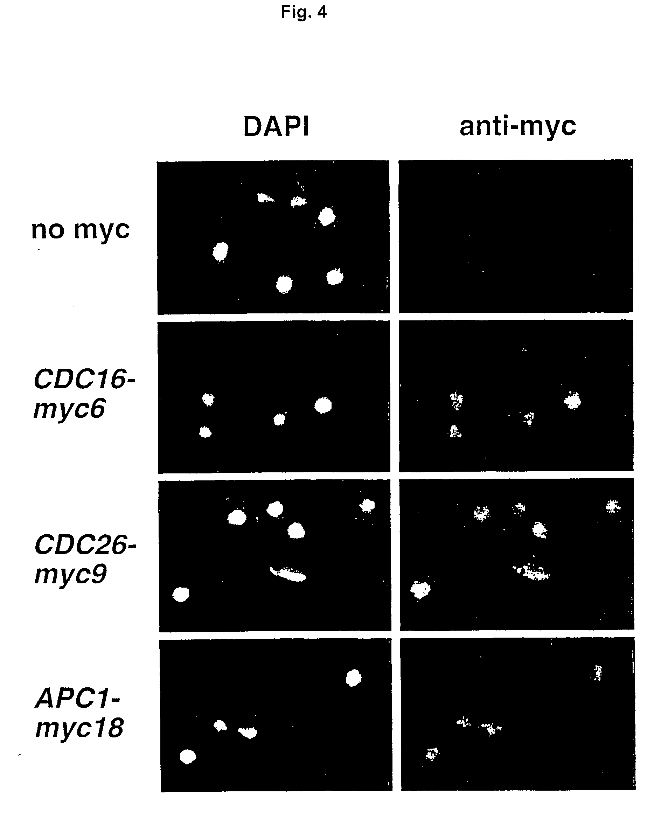

The sub-cellular localisation of the APC components was determined by indirect immunofluorescence. Cdc16-myc6p, Cdc26-myc9p, and Apc1-myc18p are localized in the nucleus (Fig.4). Cdc23-myc9p, Cdc27-myc9p and Cse1-myc9p were also found to be nuclear proteins. Specific accumulation of any of these proteins at spindle pole bodies or on mitotic spindles was not detected.

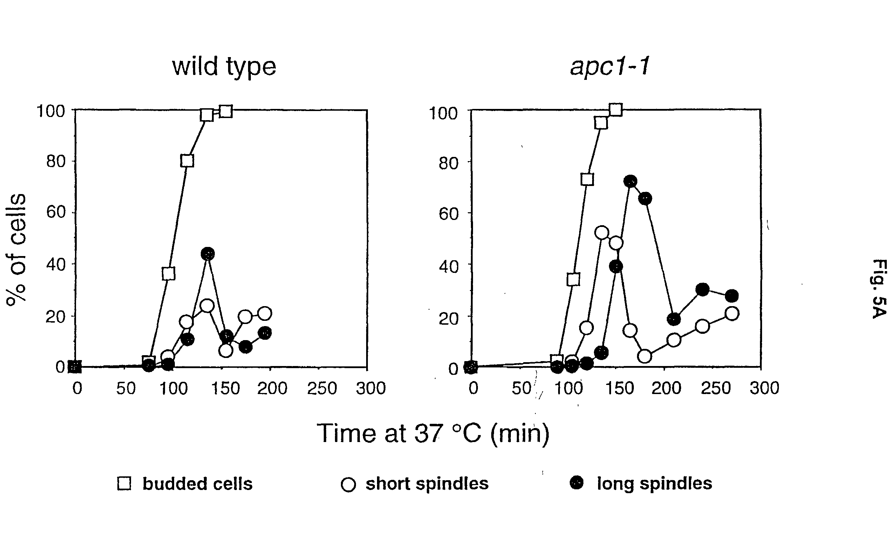

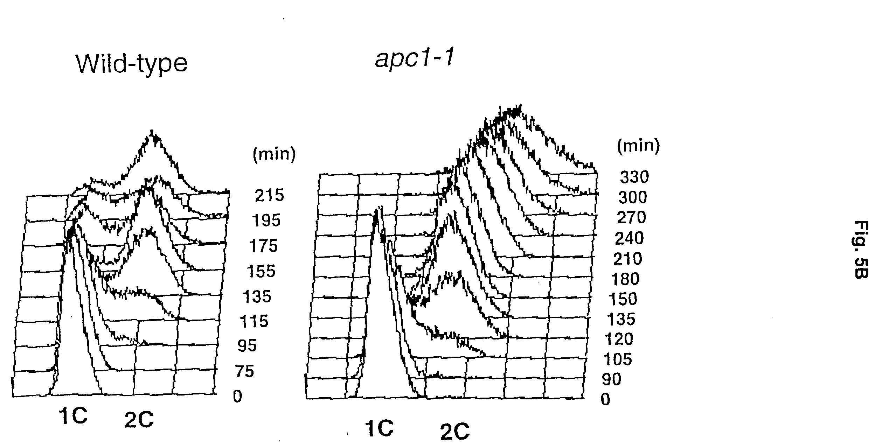

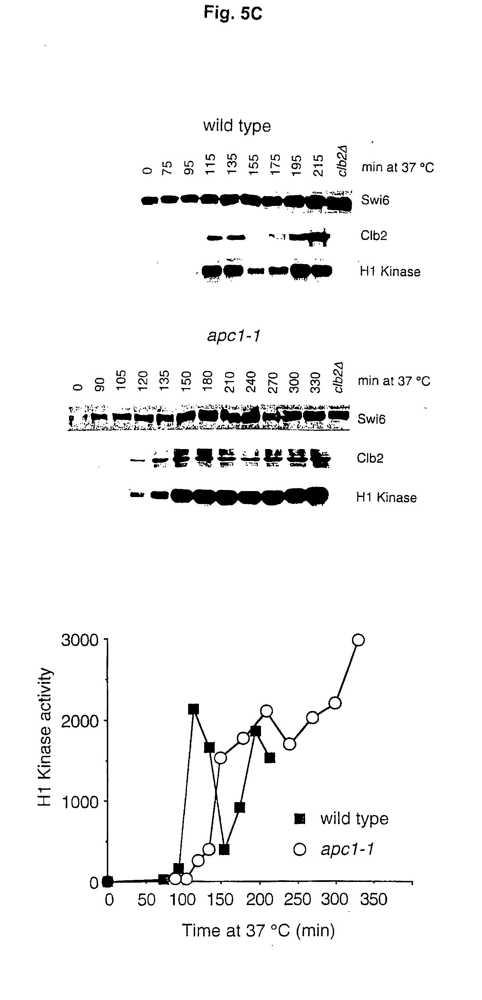

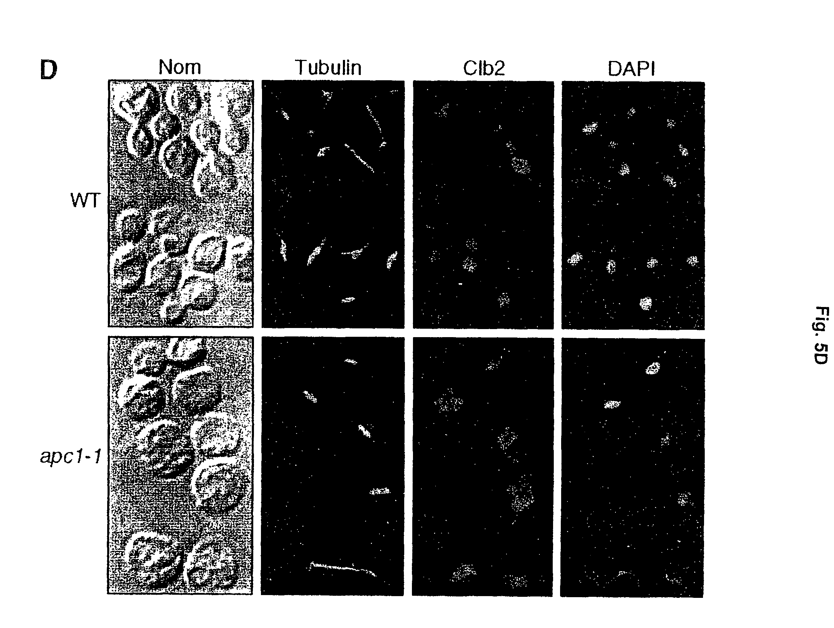

To investigate the role of *APC1* in vivo, small, unbudded G1 cells were isolated from wild-type and *apc1-1* cultures grown at 25°C, and their progression was followed through the cell cycle upon incubation at 37°C. In *apc1-1* cells DNA replication, budding, and the formation of mitotic spindles occurred at the same time as in wild-type cells but entry into anaphase was delayed by approximately 20 min (Fig. 5, A and B). Most *apc1-1* cells later underwent anaphase but were slow in disassembling their mitotic spindles. They eventually rebudded without undergoing cytokinesis or re-replication. It conclude that *apc1-1* cells are defective in the onset of anaphase, in the final exit from mitosis, and in the completion of cytokinesis. In the *apc1-1* mutant, Clb2 protein and Clb2p-associated histone H1 kinase activity appeared later than in wild-type cells. This finding could explain why entry into anaphase is delayed in the mutant. Neither Clb2 protein nor Clb2p-Cdc28p kinase activity declined as mutant cells underwent anaphase (Fig. 5C). The observation that *apc1-1* cells rebud without any apparent drop in kinase activity is surprising because high Clbp-Cdc28p kinase activity is thought to inhibit rebudding (Dahman et al., 1995).

To detect Clb2p in individual cells by indirect immunofluorescence microscopy, the endogenous *CLB2* genes of a wild-type and an *apc1-1* strain were replaced by the epitope-tagged variant *CLB2-myc12*. G1 cells, obtained by growth to stationary phase at 25°C, were inoculated into fresh medium at 37°C causing cells to re-enter the cell cycle. In wild-type cells Clb2-myc12p accumulated in maximal amounts at the onset of anaphase and then rapidly declined as cells underwent nuclear division. In the *apc1-1* mutant the amount of Clb2-myc12p remained high in cells containing separated chromosomes and fully elongated spindles (Fig. 5D). It was concluded that Apc1p is required for cyclin proteolysis not only in G1 but also in late anaphase-telophase. Surprisingly, *apc1-1* cells were defective in the formation of astral microtubules emanating from the poles of mitotic spindles (Fig. 5D). In contrast, *cdc16-123* cells arrested at 37°C had normal astral microtubules.

The accumulation of Cdc26p at high temperature is consistent with the observation that Cdc26p function is only essential for APC activity at 37°C. Cdc26p may be required to stabilize the APC or to modulate its activity under conditions of stress, such as heat shock. The discovery that the BIMEp homologs of yeast (Apc1p) is a subunits of the APC explains the pre-anaphase arrest of *bimE* mutants. Loss of *bimE* function partially bypasses the control mechanisms that render entry into mitosis dependent on the completion of DNA replication and on the activation of the NIMA kinase (Osmani et al., 1995). Taken together these data indicate that the APC is not only required for the onset of anaphase and the exit from mitosis but may also be involved in regulating entry into mitosis.

Although several subunits of the yeast APC, such as the Apc1p/BIME protein, the Cdc26 protein, and the tetratricopeptide repeat proteins Cdc16p, Cdc23p, and Cdc27p, could be identified through genetic analysis, purification of the APC from both yeast and *Xenopus* eggs showed that it contains several additional subunits. Sequencing of the entire yeast genome and recent advances in the analysis of proteins by mass spectrometry opens up an entirely new avenue for identifying and characterising the protein components of multisubunit complexes (Neubauer et al., 1997; Lamond and Mann, 1997). In the first phase, "forward" genetics is used to identify one or more components, which are then tagged with epitopes by which the entire complex can be purified by immunoprecipitation. In the second phase, the remaining unknown subunits are identified by mass spectrometry and their biological role characterized by "reverse" genetics. Using this approach, five additional subunits of the yeast APC have been identified and characterized in experiments of the present invention.

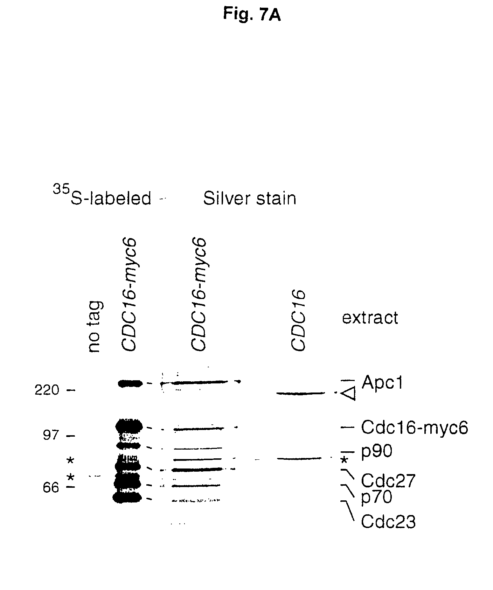

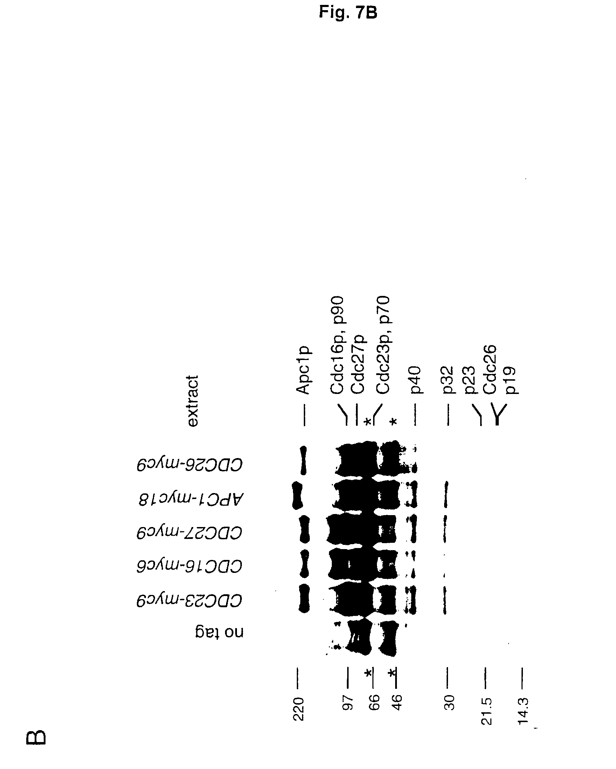

To analyse the composition of the APC, *CDC16-myc6* cells were labeled with ³⁵S and the complex was immunoprecipitated with an antibody to the myc epitope. Proteins of approximately 90 and 70 kD were detected in addition to Apc1p, Cdc16-myc6p, Cdc27p and Cdc23p (Fig. 7A). Mass spectrometric analysis revealed that the 70 kD band consists of two proteins, p70 and p68 (see below). In the low molecular weight range, proteins migrating at approximately 40, 32, 23, and 19 kD were detected (Fig.7B). All of these proteins were detected in precipitates from strains expressing different epitope-tagged APC subunits but not from wild type strains or strains expressing Cse1-myc9p which is not associated with the APC. These data suggest that the yeast APC contains at least 12 different subunits.

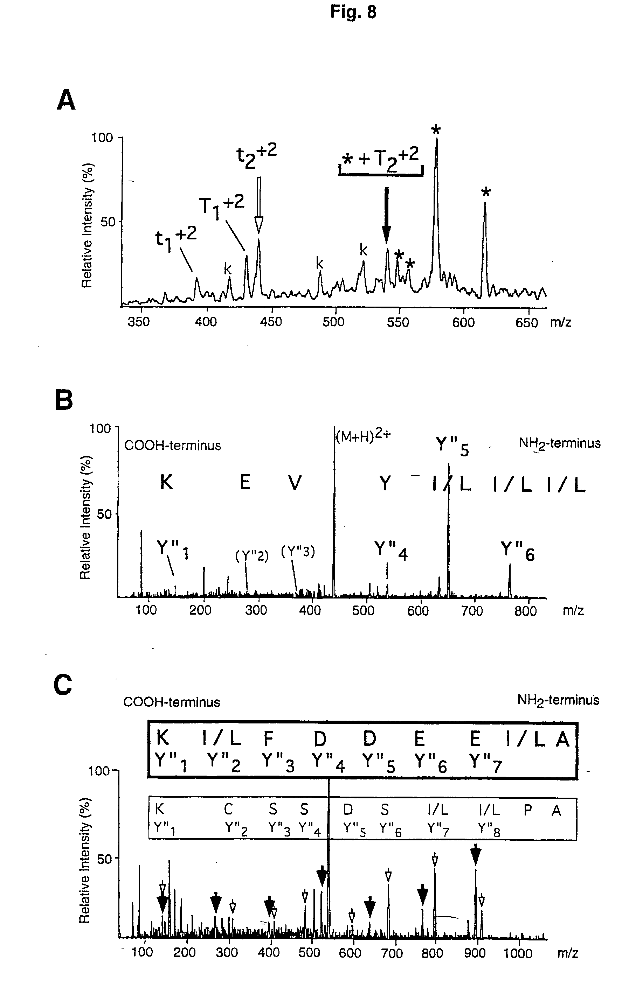

To identify these proteins, the APC was purified from *CDC16-myc6* or *CDC23-myc9* strains. Proteins were identified by mass spectrometric analysis using a previously developed two-step strategy (Shevchenko et al., 1996 a)). The mass spectrometric analysis led to the identification of p90, p70, p68, p40, and p23 as the gene products of the open reading frames YLR127c [*APC2*, 853 amino acids (aa)], YOR249c (*APC5*, 685 aa), YDR118w (*APC4*, 652 aa), YLR102c (*APC9*, 265 aa) and YDL008w (*APC11*, 165 aa), respectively (Fig. 8).

The identity of these proteins as APC subunits was confirmed by modifying the endogenous genes to encode epitope-tagged variants.

As a first step towards analysing the function of these genes, one copy of each open reading frame in diploid strains was replaced by a *HIS* marker which complements the *his3* mutation. Tetrad analysis revealed that *APC2, APC4, APC5,* and *APC11* are essential genes.

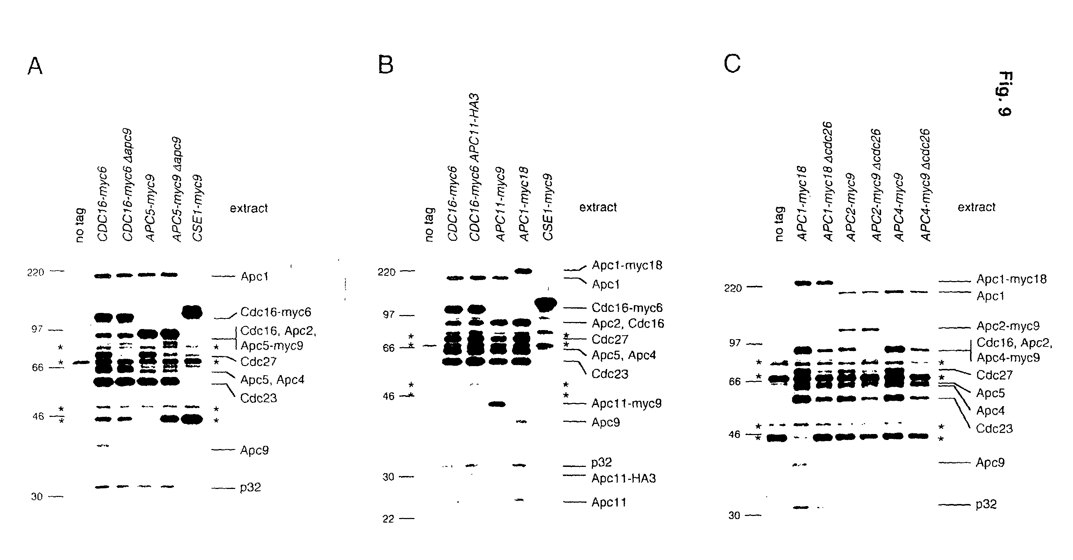

Haploid cells containing a deletion of *APC9* were viable at 25 and 37°C. It was found, however, that Cdc27p was largely absent in precipitates from *apc9* strains (Fig. 9A). Apc9p might therefore be required to stabilise the interaction of Cdc27p with the APC. To analyse the function of Apc9p in vivo, small unbudded cells from a *apc9* strain were isolated and their progression through the cell cycle upon incubation at 37°C was followed. *apc9* cells showed a defect in the onset of anaphase as indicated by the accumulation of cells with short spindles and a delay by approximately 15 min of spindle elongation and sister chromatid separation. Exit from mitosis and cytokinesis seemed to occur normally. Although not an essential gene, *APC9* is clearly required for efficient entry into anaphase. Apc9p is not the only non-essential APC subunit. *CDC26* encodes a small, heat-inducible subunit which is only essential at increased temperatures. In immunoprecipitates from *Δcdc26* strains, the amounts of Cdc16p, Cdc27p and Apc9p were severely reduced whereas the other subunits were still found to be associated with each other (Fig.9C). These data suggest that Cdc26p is required for the incorporation of a set of subunits into the APC, especially at increased temperature.

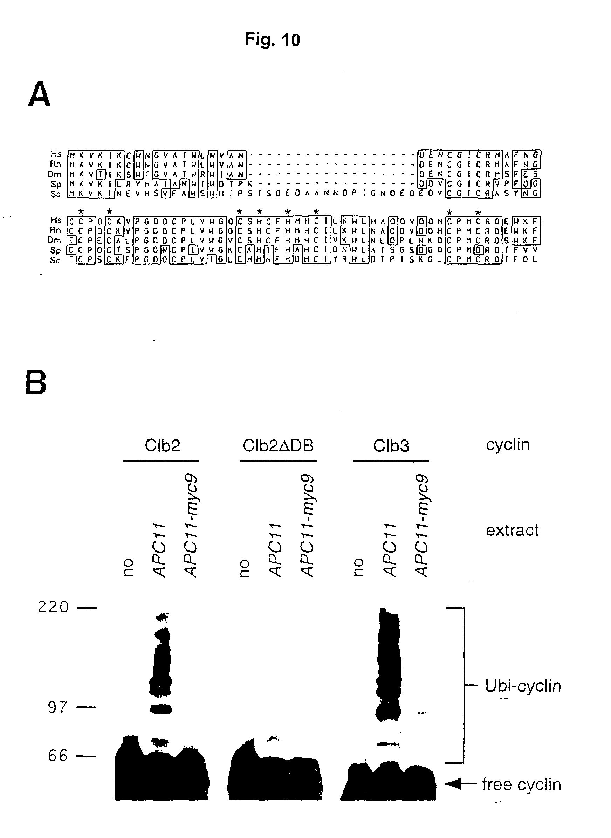

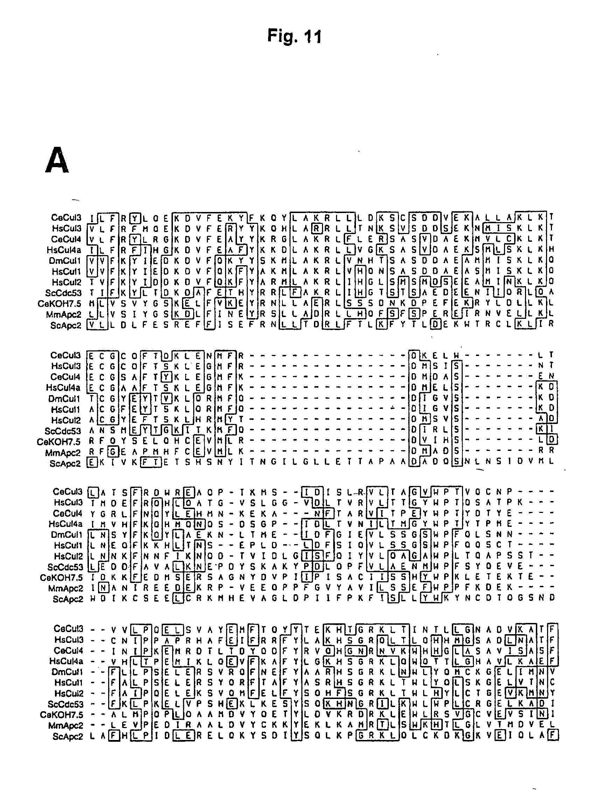

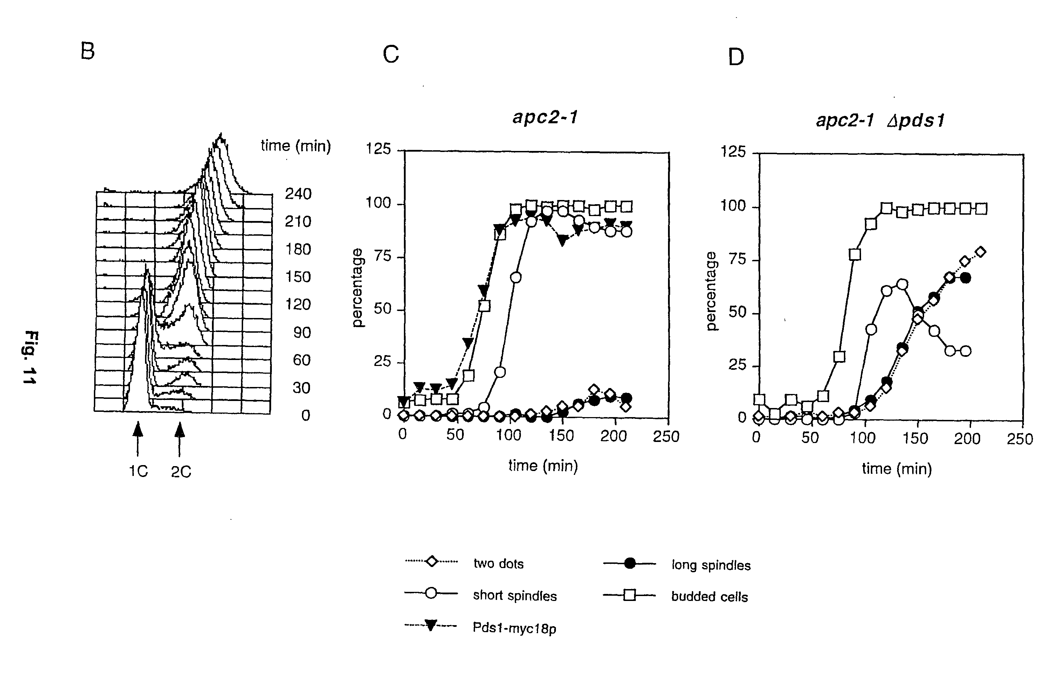

Database searches identified Apc11p as a member of a highly conserved family of proteins containing a RING-H2 domain, a variant of the RING finger (Fig. 10A). The RING domain contains two zinc ions, is found in many eukaryotic proteins with diverse and seemingly unrelated functions, and is thought to mediate protein-protein interactions (Saurin et al., 1996; Borden and Freemont, 1996).

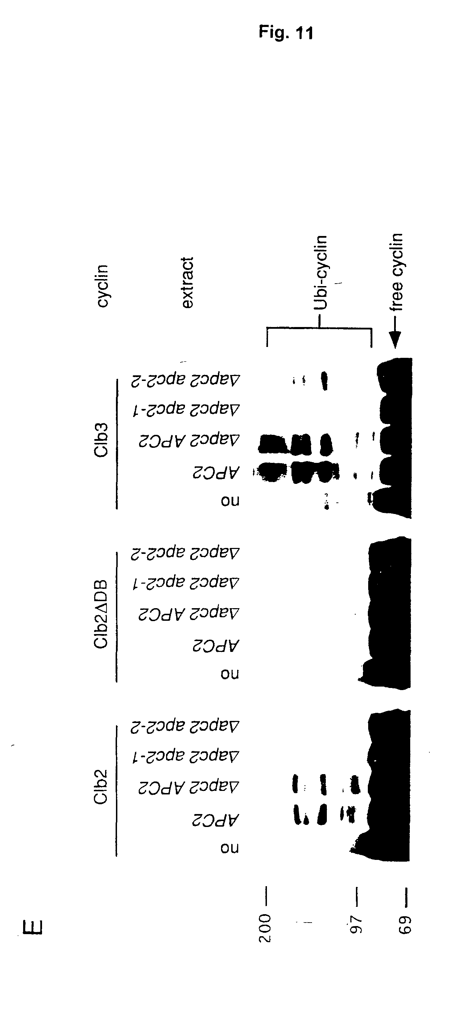

Database searches revealed homology of the COOH-terminal region of Apc2p to a putative open reading frame from *C. elegans* (KO6H7.5) and to a mouse protein whose C-terminal 339 amino acids could be assembled from Expressed Sequence Tags (ESTs) (Fig. 11A). The mouse sequence is 96% identical to that of the human Apc2 protein. Apc2p contains a domain of 180 aa which is homologous to a family of related proteins called cullins including the yeast Cdc53 protein (Fig. 11A). Apc2p and Cdc53p may therefore perform related functions in the two distinct ubiquitin-protein ligase complexes.

The analysis of Apc2p has shown that it is an essential subunit of the yeast APC. APC2 has human and mouse homologues and shows homology to cullins including yeast CDC53. Apc2p is essential for entry into anaphase and is required for ubiquitination and subsequent degradation of mitotic B-type cyclins. The only essential function of Apc2 (and the entire APC) with respect to anaphase onset is the ubiquitination of Pds1 which causes its degradation. Deletion of PDS1 caused apc2 mutant cells to undergo anaphase (Fig. 11D).

The yeast Apc5p sequence shows homology to the human Apc5. The yeast Apc4p sequence shows weak homology to the human Apc4p sequence and to the open reading frame Z97209 from *S. pombe* which is more closely related to the human protein. Apc4p might represent an APC component which has diverged more during evolution than the other subunits. No homologues have been identified for Apc9p. The data obtained in the present invention therefore suggest that, in addition to Apc1p, Cdc16p, Cdc27p, and Cdc23p, at least Apc2p, Apc5p, and Apc11p are conserved subunits of the APC in all eukaryotes. The homology between Apc2p and the cullin Cdc53p points to a common function of these proteins in the APC and the SPC. The cullin homology domain may be required for the interaction with a component that is commonly required by different ubiquitin-protein ligases. Ubiquitin-conjugating enzymes which all share a conserved domain and ubiquitin are obvious candidates. The methods used in the present invention to analyse the composition of the APC may represent a general strategy to assign a function to many uncharacterised open reading frames provided by the complete genome sequences of model organisms such as yeast.

### Brief description of the figures:

- Fig. 1A:: Accumulation of Clb2p in G1-arrested *apc1-1* cells. Immunoblot and DNA content at different time points
- Fig. 1B:: Extracts from G1-arrested *cdc26-519* cells are defective in the ubiquitination of mitotic cyclins. Immunoblot of reactions with extracts from G1-arrested wild type and *cdc26-519* cells.
- Fig. 1C:: Extracts from G1-arrested *apc1-1* cells are defective in the ubiquitination of mitotic cyclins. Immunoblot.
- Fig. 2A: Co-immmunoprecipitation of Apc1p and Cdc26p with other subunits of the APC.
- Fig. 2B:: Precipitation of the APC from metabolically labeled cells
- Fig. 3A:: Determination of the size of the yeast APC. Apc1p and Cdc16p co-sediment as a particle of 36S.
- Fig. 3B:: Determination of the size of the yeast APC. Cdc26p co-sediments with Cdc16p as a 36S particle. Cselp does not co-sediment with the APC.
- Fig. 4:: Nuclear localisation of subunits of the yeast APC. Detection of Cdc16-myc6p, Cdc26-myc9p, and Apc1-myc18p by indirect immunofluorescence.
- Fig. 5A:: Role of *APC1 in vivo.* Defective anaphase in the *apc1-1* mutant.
- Fig. 5B:: Role of *APC1 in vivo.* Distribution of the DNA content. Comparison of DNA replication in *apc1-1* cells and in wild type cells.
- Fig. 5C:: Role of *APC1 in vivo.* Clb2 protein and Clb2p-associated kinase activity.
- Fig. 5D:: Role of *APC1 in vivo.* Defective Clb2p destruction and lack of astral microtubules in *apc1-1* cells.
- Fig. 6A:: Apc1p, BIMEp and Tsg24p belong to a family of homologous proteins. Dot blot comparison.
- Fig. 6B:: Sequence alignment of the C-terminal homologous regions of Apc1p, BIMEp and Tsg24p.
- Fig. 7A:: Detection and purification of APC subunits in the high molecular weight range.
- Fig. 7B:: APC subunits in the low-molecular weight range.
- Fig. 8A:: Mass spectrum of the unseparated tryptic peptide mixture from band p70 acquired in parent ion scan mode.
- Fig. 8B:: Identification of Apc4p from the tandem mass spectrum of the doubly charged ion t₂ with a mass-to-charge ratio (m/z) of 439.2.
- Fig. 8C:: Identification of a second protein (Apc5p) in the 70 kD band.
- Fig. 9A:: Co-precipitation of APC subunits with Apc5-myc9p and subunit composition of the APC isolated from *Δapc9* strains.
- Fig. 9B:: Identification of Apc11p as an APC subunit.
- Fig. 9C:: Co-precipitation of APC subunits with Apc2-myc9p and Apc4-myc9p and subunit composition of the APC isolated from *Δcdc26* strains.
- Fig. 10A:: Homology of Apc11p to a family of RING finger proteins.
- Fig. 10B:: Defective cyclin ubiquitination in extracts from *APC11-myc9* cells.
- Fig. 11A:: A domain with homology to cullins in Apc2p.
- Fig. 11B:: Distribution of the cellular DNA content in *apc2-1* cells at the restrictive temperature.
- Fig. 11C:: Percentage of cells with buds, short spindles, long spindles, separated sister chromatids after release of small G₁ cells at 37 °C of an *apc2-1* strain containing the tetO tetR-GFP system.
- Fig. 11D:: Release of small G₁ cells of an *apc2-1 Δpds1* strain containing the tetO tetR-GFP system.
- Fig. 11E:: Ubiquitination of mitotic cyclins in extracts from wild type (*APC2*), *Δapc2 APC2, Δapc2 apc2-1* and *Δapc2 apc2-2* cells.

In the following examples, unless stated otherwise, standard methods for DNA manipulation were used as described by Ausubel et al., 1994. Unless stated otherwise, manipulation of yeast was carried out as described by Guthrie and Fink, 1991.

### Example 1

### Isolation of mutants defective in cyclin degradation and cloning of the respective genes

Mutants defective in the proteolysis of the mitotic cyclin Clb2p were isolated as described recently (Irninger, et al., 1995). Briefly, a *Δcln1,2,3 MET3* promoter-*CLN2 GAL1-10* promoter-*CLB2-lacZ* strain (K3828) was mutagenized with EMS. Cells were first arrested in G1 and then induced to express a Clb2-lacZ protein at 37°C by sequentially transferring colonies to medium containing methionine and to medium containing methionine plus galactose, respectively. Colonies (400,000) were screened for beta-galactosidase activity due to the accumulation of the Clb2-lacZ protein. Potential mutants were grown at 25°C and subsequently shifted to 37°C. The cellular DNA content was analysed by flow cytometry and mutants that arrested as large budded cells with a 2C DNA content were further analysed. The original mutants were back-crossed at least three times to wild type.

Complementation analysis and gene cloning demonstrated that new alleles of *CSE1* (7 alleles), *CDC16* (4 alleles), *CDC26 (cdc26-519)* and a mutant allele of a new gene had been isolated. The new gene was called *APC1*. For gene cloning, mutants were transformed with a genomic library (Cvrckova and Nasmyth, 1993) and plasmids allowing growth at 37°C were recovered. The temperature sensitive lethality of mutant 519 was complemented by the *CDC26* gene (Akari et al., 1992) present on a plasmid containing sequences from chromosome VI. *A LEU2* marker was integrated at the genomic *CDC26* locus and the resulting *CDC26::LEU2* strain was crossed to a *cdc26-519* mutantAll 17 tetrads derived from this cross were parental ditypes demonstrating that the cloned gene is closely linked to the *cdc26-519* mutation.

The *apc1-1* mutation was complemented by the open reading frame YNL172W (*Saccharomyces* Genome Database) present on two plasmids containing different but overlapping sequences from chromosome XTV. A *HIS3* marker was integrated at the genomic *APC1* locus and the resulting *APC1-HA3-HIS3* strain was crossed to an *apc1-1* mutant. All 38 tetrads were parental ditypes indicating that the cloned gene is closely linked to the *apc1-1* mutation.

The *APC1* wild-type gene encodes a 1748-amino acid (192-kD) protein whose COOH-terminal half is similar to the BIME protein from *A. nidulans* (Engle et al., 1990) and to a related protein encoded by the mouse *tsg24* gene (Starborg et al., 1994).

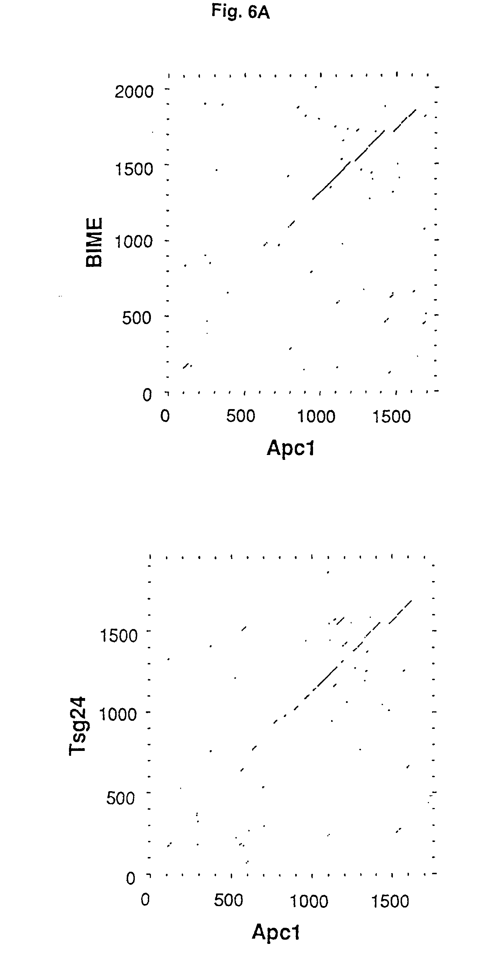

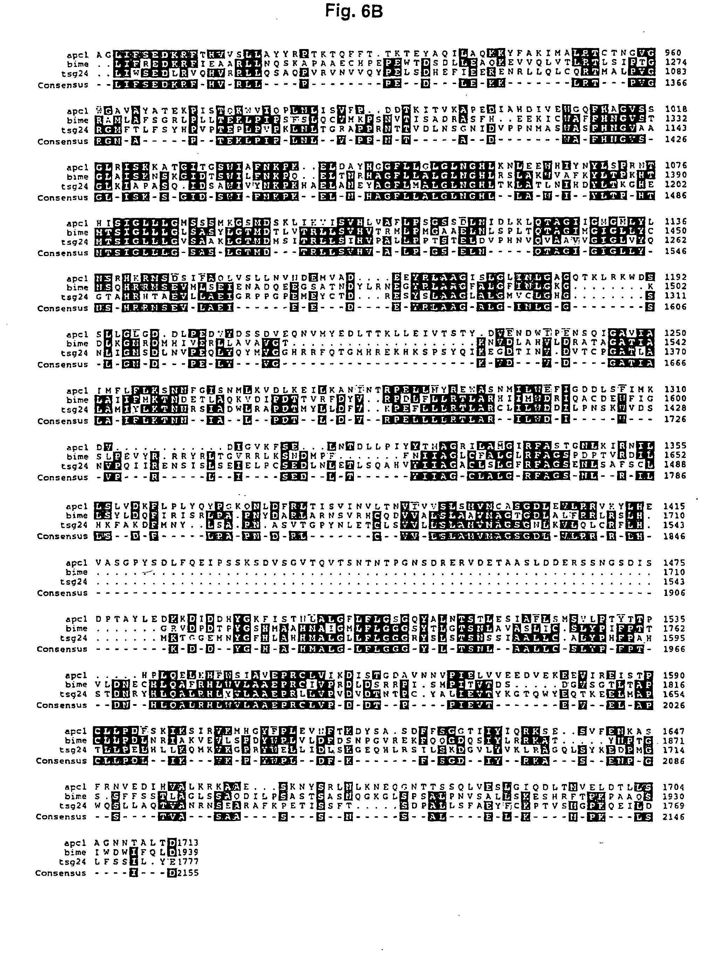

Fig. 6 shows that Apc1p, BIMEp and Tsg24p belong to a family of homologous proteins. Fig. 6A shows a dot blot comparison of Apc1p with BIMEp (top) and Apc1p with Tsg24p (bottom). The COMPARE program (Window 50, Stringency 23) of the GCG software package was used for sequence comparison.

Fig. 6B shows the sequence alignment of the C-terminal homologous regions of Apc1p, BIMEp and Tsg24p. Identical amino acids are indicated with black boxes. The PILEUP program of the GCG software was used.

### Example 2

### Defective proteolysis and ubiquitination of mitotic cyclins in the cdc26-519 and apc1-1 mutants

### A: Accumulation of Clb2p in G1-arrested apc1-1 cells

Wild-type (K5137) and *apc1-1* cells (K6221) of the genotype *MATa GALp-CLB2-HA3 bar1* were grown at 23°C in 400 ml YEP medium (1% yeast extract, 1% peptone) containing 2% raffinose to a cell density of 0.3 x 10⁷ cells/ml. Cells were arrested in G1 by addition of alpha-factor (0.5 µg/ml) to the medium for 5 h. At time point 0, cells were transferred to YEP medium containing 2% raffinose plus 2% galactose and alpha-factor (0.5 µg/ml) at 35°C. Samples for immuno-blotting (40 ml culture) and flow cytometric analysis of cellular DNA content (2 ml culture) were withdrawn at the indicated time points. Protein extracts were prepared and Clb2-HA3p was detected by immuno-blotting using the monoclonal antibody 12CA5 directed against the HA epitope tag. Cdc28p was detected as a loading control using a rabbit antiserum. Preparation of protein extracts and immunoblotting were carried out as described (Surana, et al., 1993). Bands were detected with the enhanced chemiluminescence system (ECL, Amersham). A Becton-Dickinson FACScan was used for analysis of cellular DNA content (Epstein and Cross, 1992). Fig. 1A shows the immunoblot and the DNA content at different time points. Clb2-HA3p accumulated in the *apc1-1* cells but not in wild type cells. *apc1-1* cells initiated DNA replication whereas wild type cells stayed arrested.

### B: Extracts from G1-arrested cdc26-519 cells are defective in the ubiquitination of mitotic cyclins

A wild type (K5518) and two *cdc26-519* strains (K6200 and YWZ135) (all strains are *MATa pep4 bar1)* were grown at 25°C in 300 ml YEP medium containing 2% raffinose and 50 mM sodium phosphate pH 6.5 to a cell density of 0.55 x 10⁷ cells/ml. Cells were arrested in G1 with alpha-factor (0.5 µg/ml) for 2.5 h at 25°C and then shifted to 37°C for 1.5 h.

Protein extracts for the ubiquitination assay and the ubiquitination reaction were prepared essentially as described (Zachariae and Nasmyth, 1996). Cells were collected on membrane filters and incubated in 30 ml of Tris-SO₄/DTT solution or 3 min. Cells were washed with 30 ml spheroplast medium containing 0.5% raffinose and 0.5 µg/ml alpha-factor (pre-warmed to 37°C) and incubated in the same medium containing 0.5 mg lyticase (Sigma, L5763) for 10 min at 37°C. The following steps were carried out at 0 to 4 °C. The spheroplast suspension was diluted to 50 ml with ice cold 1.2 M sorbitol. Spheroplasts were collected by centrifugation, resuspended in 50 ml sorbitol and collected again. The spheroplasts were resuspended in 0.2 ml of buffer A and disrupted by shaking with 0.3 ml of glass beads (0.5 mm) for 4 min. The lysate was cleared by centrifugation for 5 min in an Eppendorf micro-centrifuge and the supernatant was used directly for the ubiquitination assay. Protein concentrations were determined with the Bio-Rad protein assay using bovine serum albumin as a standard. The protein concentrations of the extracts used for this experiment were 50 mg/ml.

Extracts containing the cyclin-substrates were prepared as described above from strains overexpressing HA3-tagged Clb2p, Clb2p with a deletion of the destruction box (Clb2ΔDBp) (Zachariae and Nasmyth, 1996), and Clb3p. Extracts were frozen in aliquots (20 µl) in liquid nitrogen and stored at -80°C. The protein concentration of these extracts was 40 mg/ml.

32 µl extract was mixed with 4 µl ATP-regenerating system in a total volume of 38 µl. 2 µl of extract containing the cyclin substrates Clb2-HA3p, Clb2ΔDB-HA3p or Clb3-HA3p was added and the reaction was incubated for 5 min at room temperature. Reactions were terminated by diluting samples 1:5 into hot SDS sample buffer. 25 µl samples were separated by electrophoresis on 8 % SDS-polyacrylamide gels and subsequently blotted to a membrane. HA-tagged cyclin and cyclin-ubiquitin conjugates were detected with the monoclonal antibody 12CA5 and an enhanced chemoluminescence detection system purchased from Amersham.

Fig. 1B shows the immunoblot of reactions with extracts from G1-arrested wild type and *cdc26-519* cells. The wild type extract conjugates ubiquitin (which is present in the extract in sufficient amounts) to Clb2-HA3p and Clb3-HA3p but not to C1b2ΔDB-HA3p. The extracts from the mutant strains are defective in ubiquitination of Clb2-HA3p and Clb3-HA3p.

### C: Extracts from G1-arrested apc1-1 cells are defective in the ubiquitination of mitotic cyclins

A wild type (K1771) and an *apc1-1* strain (K6199) (both strains have the genotype *MATa pep4 bar1)* were grown at 23°C in 300 ml YEP medium containing 2 % raffinose and 50 mM sodium phosphate pH 6.5 to a cell density of 0.55 x 10⁷ cells/ml. Cells were arrested in G1 with alpha-factor (0.5 µg/ml) for 4.5 h at 23°C and then shifted to 37°C for 40 min. The G1-arrest was confermed by flow cytometric analysis of the cellular DNA content. Protein extracts were prepared and used for ubiquitination reactions as described above. The protein concentrations of the extracts used in this experiment were 30 mg/ml. Fig. 1C shows the immunoblot of the reactions. The extract from the *apc1-1* mutant is defective in the ubiquitination of the cyclin substrates Clb2-HA3p and Clb3-HA3p.

### Example 3

### Disruption of the APC1 and CDC26 genes

### A: APC1 is an essential gene

A plasmid was constructed in which a 4.3-kb Bam HI to Sty I fragment within the APC1 gene was replaced by *HIS3*. The *apc1::HIS3* construct was transformed into a diploid wild type strain. Clones in which one *APC1* allele was disrupted by *HIS3* were identified by Southern (DNA) blot analysis of genomic DNA isolated from His⁺ transformants. An *apc1::HIS3*/*APC1* strain was sporulated and tetrads were dissected. His⁺ spores arrested as large budded cells after one or two cell divisions after germination whereas His- spores grew normally. These data show that *APC1* is an essential gene.

### B: CDC26 is only essential at high temperature

A method using fragments generated by polymerase chain reaction (PCR) to disrupt genes with the *URA3* gene from *Kluyveromyces lactis* is described by Längle-Rouault and Jacobs, 1995.

A fragment was amplified by polymerase chain reaction (PCR) containing the *Kluyveromyces lactis URA3* gene flanked by two *CDC26* sequences of 45 bp which are located upstream and downstream of the start and stop codon, respectively. The PCR fragment was transformed into a diploid wild type strain. Clones in which one *CDC26* allele was replaced by the *K. lactis URA3* gene were identified by PCR-amplification of the *CDC26* locus form genomic DNA isolated from Ura⁺ transformants. A *Δcdc26::KlURA3*/*CDC26* strain was sporulated and tetrads were dissected. Tetrads consisted of two Ura⁺ spores which were viable at 25°C but arrested as large budded cells at 37°C and two Ura⁻ spores which grew normally at 37°C. These data show that *CDC26* is only essential for proliferation at 37°C.

### Example 4

### Analysis of the subunit composition of the APC

### A: Epitope tagging

To investigate whether Cdc26p, Apc1p, and Cselp associate with the Cdc16p-Cdc23p-Cdc27p complex, the endogenous genes were modified to encode variants carrying COOH-terminal HA or myc epitope tags: Two fragment emcopassing 200 bp of coding region upstream of the stop codon and 200 bp of 3'-non-coding region downstream of the stop codon, respectively, were amplified by PCR. The PCR-primers were choosen to introduce an artificial restriction site in front of the stop codon. The two fragments were ligated into a yeast integrative vector and fragments encoding several HA (three HA epitopes) or myc epitope tags (six or nine myc epitopes) were inserted into the restriction site in front of the stop codon. The following yeast integrative vectors were used: YIplac128 *(LEU2),* YIplac204 (*TRP1*), YIplac211 (*URA3*) (Gietz and Sugino, 1988); pRS303 (*HIS3*), (Sikorski and Hieter, 1989). The resulting plasmid was transformed into yeast. Homologous recombination into the respective genomic locus generates an epitope-tagged full-length version of the gene linked to a selectable marker. Epitope-tagged *CDC16, CDC23* and *CDC27* were marked with *URA3, LEU2* and *TRP1*, respectively. Tagged *CDC26, APC1* and *CSE1* were each marked with *HIS3.* Homologous integration of the plasmid was verified by Southern (DNA) blot analysis and detection of the epitope tagged protein by immunoblotting using anti-HA or anti-myc antibodies. Strains expressing two epitope-tagged proteins were obtained by genetic crosses. At 37°C all strains grew normally demonstrating that the epitope-tagged proteins were fully functional.

### B: Co-immmunoprecipitation of Apc1p and Cdc26p with other subunits of the APC

Extracts were prepared from strains expressing a HA-tagged protein and a myc-tagged protein (e. g. an *APC1-HA3 CDC16-myc6* strain). Extracts were subjected to immunoprecipitations with the antibody to HA and co-precipitation of the myc-tagged protein was analysed by immunoblotting. Extracts prepared from strains expressing only the respective myc-tagged protein (e. g. a *CDC16-myc6* strain) were used as controls.

Extracts for immunoprecipitations were prepared as described (Lamb, et al., 1994). Strains were grown at 30°C in YEP medium (1% yeast extract, 1% peptone) containing 2% glucose to a cell density of 0.7 x 10⁷ cells/ml. 2 x 10⁹ cells were lysed with glass beads in 0.4 ml buffer A (50 mM Tris-HCl pH 7.5, 50 mM NaCl, 0.2% Triton X100, 10% glycerol, 1 mM DTT, 1 mM PMSF, 1 µg/ml of leupeptin and pepstatin). Extracts were cleared by centrifugation (20 min 12,000 x g). 0.3 ml extract adjusted to a protein concentration of 20 mg/ml was incubated for 1 h at 4°C with 30 µl of a 50% suspension of 12CA5 (anti-HA) antibody crosslinked to protein A-Sepharose. Crosslinking was carried out as described by Harlow and Lane, 1988. The beads were washed three times with 1 ml of buffer A and bound proteins were released by addition of SDS to 3%. After removal of the beads by filtration, samples were analyzed by immuno-blotting using anti-HA (12CA5) or anti-myc (9E10) monoclonal antibodies.

Immunoblots of co-precipitation experiments are shown in Fig. 2A. Cdc16-myc6p was co-precipitated with Apc1-HA3p. Cdc26-myc9p was co-precipitated with Cdc16-HA3p. Apcl-myc6p was co-precipitated with Apc1-HA3p from an extract prepared from an *APC1-HA3*/*APC1-myc6* diploid strain. These data together with previous work (Zachariae and Nasmyth, 1996; Lamb, et al., 1994) suggest that cyclin ubiquitination in yeast depends on a complex containing at least two molecules each of Apc1p, Cdc16p, Cdc23p, and Cdc27p. Cdc26p is also components of this complex.

### C: Precipitation of the APC from metabolically labeled cells

To characterize the complex further, various strains, each expressing a different myc-tagged protein, were labeled with ³⁵S methionine and cysteine. Extracts were prepared and subjected to immunoprecipitations using the antibody to the myc epitope tag. Precipitated proteins were separated by SDS-polyacrylamide gelelectrophoresis and detected by autoradiography.

Strains were grown at 30°C in synthetic complete medium lacking methionine. 5 x 10⁷ cells were labeled with 1 mCi ³⁵S methionine/cysteine in 1.5 ml medium for 2 hours at 30°C (Sherman, 1991). Cells were broken with glass-beads in 0.25 ml buffer B [buffer A (Zachariae and Nasmyth, 1996) diluted 1:3] containing 1.5 mg unlabeled protein from a *pep4* strain (K5517) (Zachariae and Nasmyth, 1996). After centrifugation (10 min, 12,000 g), extracts were sequentially incubated with protein A-sepharose (160 µl) and 9E10 antibody crosslinked to protein A-sepharose (25 µl). The beads were washed with buffer B (4 x 1 ml), buffer B with 120 mM K-acetate (1 ml) and buffer B with 150 mM K-acetate (1 ml). Bound proteins were released by addition of SDS to 3%, heated in SDS-sample buffer and separated on 8 % SDS-polyacrylamide gels. Gels were treated with Entensify solution (Du Pont de Nemours), dried on filter paper and exposed to X-OMAT film (Kodak) for 12 h.

An autoradiograph is shown in Fig. 2B. The same set of proteins was precipitated from extracts of *CDC16-myc6, CDC23-myc9, CDC26-myc9, CDC27-myc9* and *APC1-myc18* cells. This set included Cdc16p, Cdc23p, Cdc27p and Apc1p, which were identified by the increased molecular weight of epitope-tagged variants, and a protein of 80 kD. A protein with a size close to that of Cdc16p (100 kD) was detected in the immunoprecipitate from the *CDC16-myc6* strain. None of these proteins were co-precipitated with Cse1-myc9p.

These data suggest that the yeast APC contains, in addition to Apc1p, Cdc16p, Cdc23p, Cdc27p, and Cdc26p, at least two unidentified proteins (p80 and p100). The Cse1 protein is not a component of the APC.

### Example 5

### Co-sedimentation of Apc1p, Cdc16p, and Cdc26p as a 36S particle

To determine the size of the yeast APC, extracts from strains expressing two epitope-tagged proteins were analysed by glycerol density gradient centrifugation.

A: An *APC1-HA3 CDC16-myc6* strain (K6190) was grown at 30°C in 200 ml of YEP medium containing 2 % raffinose to a cell density of 0.7 x 10⁷ cells/ml. Cells were broken with glass beads in 0.4 ml buffer B (50 mM Hepes-KOH pH 7.3, 60 mM K-acetate, 5 mM Mg-acetate, 0.1 % Triton X100, 1 mM DTT, 1 mM PMSF, 1µg/ml of leupeptin and pepstatin) containing 5 % glycerol. The lysate was cleared by centrifugation (20 min 12,000 x g). 0.15 ml extract (2.4 mg protein) was layered on a 10-35 % glycerol gradient in buffer B and centrifuged for 15 h at 28,000 rpm in a Beckman SW40 rotor at 4°C. 20 fractions were collected from the top and proteins were precipitated with TCA. Proteins were dissolved in SDS sample buffer, separated in 7 % SDS-polyacrylamide gels and analysed by immunoblotting. Apc1-HA3p and Cdc16-myc6p were detected with the antibodies 12CA5 and 9E10, respectively, and an enhanced chemoluminescence detection system purchased from Amersham. Cim5p, a subunit of the 19S proteasome activator complex, and fatty acid synthetase (FAS, 40.6S) were detected with polyclonal rabbit antisera. The immunoblot is shown in Fig. 3A. Apc1p and Cdc16p co-sediment as a particle of 36S.

B: Extracts from *CDC16-HA3, CSE1-myc9* (K6184) and *CDC16-HA3 CDC26-myc9* (K6323) strains were prepared and separated on parallel gradients as described in (A). The immunoblot is shown in Fig. 3B. Cdc26p co-sediments with Cdc16p as a 36S particle. Cselp does not co-sediment with the APC.

### Example 6

### Nuclear localisation of subunits of the yeast APC

Control cells (no myc, K1534) and cells containing *CDC16-myc6* (K6180), *CDC26-myc9* (K6322), or *APC1-myc18* (K6329) were grown in YEP medium containing 2 % glucose at 30°C. Cells were fixed with formaldehyde and prepared for indirect immunofluorescence. Myc-tagged proteins were detected with the 9E10 antibody and a CY3-conjugated secondary antibody. DNA was stained with DAPI. Cells were examined with a Zeiss Axiophot microscope and pictures were taken on Kodak T-MAX400 film. Cdc16-myc6p, Cdc23-myc9p, Cdc26-myc9p, Cdc27-myc9p and Apc1-myc18p are all localized in the nucleus. Cse1-myc9p was also found to be a nuclear protein. Fig. 4 shows the detection of Cdc16-myc6p, Cdc26-myc9p, and Apc1-myc18p by indirect immunofluorescence.

### Example 7

### Defective anaphase in the apc1-1 mutant

To investigate the role of *APC1 in vivo*, we isolated small, unbudded G1 cells from wild type and *apc1-1* cultures grown at 25°C and followed their progression through the cell cycle upon incubation at 37°C.
A wild-type (K699) and an *apc1-1* strain (K5717) were grown at 25°C in synthetic complete medium containing 2 % raffinose to increase the fraction of G1 cells. Small unbudded G1 cells were isolated by centrifugal elutriation as described (Schwob and Nasmyth, 1993). Small G1 cells were released into YEP medium containing 2 % glucose at 37°C and samples were withdrawn at the indicated time points. The fraction of budded cells was determined by microscopic examination of sonicated samples. Mitotic spindles were detected by indirect immunofluorescence. A Becton-Dickinson FACScan was used for flow cytometric analysis of the DNA content of cells stained with propidium iodide (Epstein and Cross, 1992). Clb2p was detected by immunoblotting using a polyclonal rabbit antiserum (Surana, et al., 1993) and an enhanced chemoluminescence detection system (ECL, Amersham). Clb2-associated Cdc28 kinase activity was detected using histone H1 as a substrate as described (Surana, et al., 1993). Kinase activities were quantified with a Molecular Dynamics PhosphorImager. A *clb2* deletion strain (K1890) was used for negative controls and Swi6p was detected as a loading control.

Strains K6208 and K6131 were constructed by replacing the endogenous *CLB2* genes of a wild type and an *apc1-1* strain, respectively, as described in example 4A. A *CLB2-myc12* strain was crossed to a *Δclb1* strain and tetrads were dissected. *Δclb1 CLB2-myc12* strains obtained in this cross grew normally at 37°C, demonstrating that the Clb2-myc12 protein was fully functional (In a *clb1* deletion strain, *CLB2* is essential for viability).

Fig. 5A shows the percentage of budded cells, of cells containing a short spindle in an undivided nucleus, and of cells with separated chromosomes and an elongated spindle (late anaphase-telophase). In *apc1-1* cells budding and the formation of mitotic spindles occured at the same time as in wild type cells but entry into anaphase was delayed by approximately 20 min. Most cells *apc1-1* cells later underwent anaphase but were slow in dissasembling their mitotic spindles. *apc1-1* cells did not undergo cytokinesis.

Fig. 5B shows the distribution of the DNA content. *apc1-1* cells initiated DNA replication at the same time as wild type cells. In contrast to wild type cells the mutant cells arrested with replicated DNA at 37°C.

Fig. 5C shows Clb2 protein and Clb2p-associated kinase activity. In *apc1-1* cells Clb2 protein and Clb2p-associated kinase activity appeared later than in wild type cells. Neither Clb2 protein nor Clb2p-Cdc28p kinase activity declined as *apc1-1* cells underwent anaphase.

Fig. 5D shows defective Clb2p destruction and lack of astral microtubules in *apc1-1* cells. A wild-type (K6208) and an *apc1-1* (K6131) strain, both containing *CLB2-myc12*, were grown for two days at 25°C on YEP-2 % glucose-plates to obtain unbudded G1 cells. Strains were inoculated into liquid medium (YEP 2 % glucose) at 37°C and samples for flow cytometric analysis of cellular DNA content and indirect immunofluorescence were taken at one-hour intervals. DNA replication and the formation of buds occured at the same time in wild type and *apc1-1* cells. Clb2-myc12p was detected with the 9E10 antibody and a CY3-conjugated secondary antibody. Spindles were detected with a rabbit antiserum to yeast tubulin and a FITC-conjugated secondary antibody. Pictures were taken with a CCD camera mounted on a Zeiss Axiophot microscope. Fig. 5D shows immunofluorescence pictures from samples taken after 3 hours. In *apc1-1* cells, Clb2-myc12p is present in high amounts in cells with fully elongated spindles (cells that underwent anaphase) whereas Clb2-myc12p is degraded during anaphase in wild type cells. *apc1-1* cells are defective in the formation of astral microtubules emanating from the poles of mitotic spindles.

### Example 8

### Analysis of the subunit composition of the APC

To analyse the composition of the APC, *CDC16-myc6* cells were labeled with ³⁵S and the complex was immunoprecipitated with an antibody to the myc epitope. Extracts were prepared in parallel from a control strain and *CDC16-myc6* or *CDC23-myc9* strains (*MATa* Δ*pep4 Δbar1*). To identify p90 and p70, cells (10¹⁰) were broken in 4 ml B70 buffer [50 mM Hepes-KOH (pH 7.3), 5 mM Mg-acetate, 0.1 % Triton X100, 20 mM β-glycero-phosphate, 10 % glycerol, 1 mM NaF, 1 mM dithiothreitol, pepstatin (1 µg/ml), proteinase inhibitors (Complete, Boehringer Mannheim), and 70 mM K-acetate]. The lysate was centrifuged twice (20 min, 18,000g). The supernatant (7 ml, 120 mg) was incubated with protein A-Sepharose (2 ml) for 1 hour, then filtered to remove the beads, centrifuged (10 min, 18,000*g*), and finally incubated with the antibody 9E10 cross-linked to protein A-Sepharose (0.2 ml) for 2.5 hours. The beads were divided into four portions which were washed with 3 x 1 ml of buffer B100, B150, and B200 (these buffers are identical with B70 except for the K-acetate concentration which was 100, 150 or 200 mM, respectively) each containing insulin (0.1 mg/ml), and 1 x 0.4 ml of buffer B containing 50 mM NaCl. Proteins were eluted with SDS (1 %) and separated on SDS-polyacrylamide gels. To identify p40 and p23, the APC was precipitated from 5.5 x 10¹⁰ cells.

Proteins of approximately 90 and 70 kD were detected in addition to Apc1p, Cdc16-myc6p, Cdc27p and Cdc23p (Fig. 7A). Mass spectrometric analysis revealed that the 70 kD band consists of two proteins, p70 and p68 (see below). In the low molecular weight range, proteins migrating at approximately 40, 32, 23, and 19 kD were detected (Fig. 7B). All of these proteins were detected in precipitates from strains expressing different epitope-tagged APC subunits but not from wild type strains or strains expressing Cse1-myc9p which is not associated with the APC.

Fig. 7 shows the analysis of the subunit composition of the APC as follows:
(A) Detection and purification of APC subunits in the high molecular weight range. Extracts were prepared from control cells or *CDC16-myc6* cells and subjected to immunoprecipitations with an antibody to the myc epitope. Bound proteins were separated on 8 % SDS polyacrylamide gels. Cells (5 x 10⁷) were labeled with ³⁵S amino acids and proteins were detected by fluorography (left panel). For mass spectrometric analysis, extracts were prepared from *Δpep4* cells (10¹⁰). Proteins were detected by silver staining according to the method described by Shevchenko et al., 1996 b) (right panel). The *CDC16* control extract contains a myc-tagged protein unrelated to APC subunits causing immunoprecipitation of a 180 kD protein (Δ). Proteins whose precipitation is not myc-dependent are marked (*). (B) APC subunits in the low-molecular weight range. Immunoprecipitates from ³⁵S-labeled cells of strains expressing different myc-tagged proteins were separated in a 4% to 20 % SDS polyacrylamide gradient gel. Proteins were detected by fluorography.

### Example 9

### Identification by nanoelectrospray tandem mass spectrometric sequencing of proteins co-purifying with the APC

To identify these proteins, the APC was purified from *CDC16-myc6* or *CDC23-myc9* strains. One-step immunoprecipitations from unfractionated whole cell extracts yielded enough material to detect individual subunits on silver-stained gels (Fig. 7A). Proteins were identified by mass spectrometric analysis using a previously developed two-step strategy (Shevchenko et al., 1996 a)). First, matrix assisted laser desorption ionization (MALDI) was used to identify proteins by generating a precise mass map of the tryptic peptides. In case this method did not provide unambiguous identification, gel pieces were extracted, and the pool of tryptic peptides analyzed by nanoelectrospray tandem mass spectrometry as described by Wilm et al., 1996 a) (see also below). Samples were sprayed for 20 minutes. Because no signals apart from trypsin autodigestion products were visible in the normal mass spectrum (Q1 scan mode), the parent ion scan mode was used which detects ions that fragment to daughter ions of m/z 86, the immonium ion of leucine and isoleucine (Wilm et al., 1996 b)). During spraying, peptide ions were selected by the first quadrupole lens of the mass spectrometer, fragmented in turn in the collision chamber and tandem mass spectra were recorded. Peptide sequence tags were derived from these spectra and searched against a comprehensive protein database using the program PeptideSearch (Mann and Wilm, 1996). Based on a BSA standard the amount of protein available for mass spectrometric identification was well below 50 ng per band.

As an example, the identification of two proteins in the 70 kD band is shown in Fig. 8. Due to the low amount of protein and the relatively crude preparation, MALDI (0.5 µl of the protein, in-gel digested with trypsin was used) did not result in useful data and a normal nanoelectrospray mass spectrum similarly did not provide clear peptide candidates. Parent ion scans were used to distinguish peptide ions from chemical noise (Fig. 8A). It was not possible to substract protein contaminations such as keratins and trypsin autolysis products at these low levels. Instead, the identification became only possible by fragmenting all peptide ions, leading mainly to keratin identification but also to four peptides from two different proteins (Fig. 8B,C). One of the peptides was identified in a mixture with a trypsin peptide (Fig. 8C). Therefore, the actual sensitivity was found to depend less on the absolute amount of proteins present but rather on the ability to sequence peptides from the target protein among the much larger number of background peptides. The mass spectrometric analysis led to the identification of p90, p70, p68, p40, and p23 as the gene products of the open reading frames (Designations of open reading frames in the *Saccharomyces* genome database are given. Yeast genes encoding homologous subunits were named according to the human APC subunits.) YLR127c [*APC2*, 853 amino acids (aa)], YOR249c (*APC5*, 685 aa), YDR118w (*APC4*, 652 aa), YLR102c (*APC9*, 265 aa) and YDL008w (*APC11*, 165 aa), respectively (Fig. 8).

Fig. 8 shows the identification by nanoelectrospray tandem mass spectrometric sequencing of proteins co-purifying with the APC as follows: (A) Mass spectrum of the unseparated tryptic peptide mixture from band p70 acquired in parent ion scan mode. This mode detects ions that fragment to daughter ions of m/z 86, the immonium ion of leucine and isoleucine (Wilm et al 1996 b). During spraying, peptide ions were selected by the first quadrupole lens of the mass spectrometer, fragmented in turn in the collision chamber and tandem mass spectra were recorded. Peptide sequence tags were derived from these spectra and searched against a comprehensive protein database using the program PeptideSearch (Mann and Wilm, 1996). Based on a BSA standard the amount of protein available for mass spectrometric identification was well below 50 ng per band. Tandem mass spectra were obtained from all labeled peaks. Upon database searching, peaks were identified as trypsin autodigestion products (*), peptides from human keratins (k), peptides derived from Apc5p (T₁ corresponds to peptide CVILLLK; T₂: ALEEDDFLK), and peptides from Apc4p (t₁: LAVIPIR; t₂: IIIYVEK). (B) Identification of Apc4p from the tandem mass spectrum of the doubly charged ion t₂ with a mass-to-charge ratio (m/z) of 439.2 (unfilled arrow in A). Collisional fragmentation of tryptic peptides produces mainly ions containing the COOH-terminal part of the peptide (Y" ions). The part of the tandem mass spectrum above the parent ion was acquired separately (31 scans accumulated) and then combined with the full scan spectrum (8 scans accumulated). This improved the signal to noise ratio in the most informative part of the spectrum which is essentially free from chemical noise. Peptide sequence tags were derived from the mass differences between adjacent Y" ions and used in database searches. After retrieving the matching sequence, all other ions (including ions with masses less than the mass of the parent ion which were largely obscured by chemical noise) were used to confirm the hit. (C) Identification of a second protein (Apc5p) in the 70 kD band. The tandem mass spectrum of the ion with m/z 539.8 (filled arrow in A) yields an ion series (filled arrow heads) from an Apc5p peptide (upper sequence) and another, non-overlapping, Y" series (unfilled arrow heads) originating from a trypsin peptide (lower sequence; C, cysteine S-acetamide).

### Example 10

### Characterisation of the identified proteins as APC subunits

The identity the proteins as APC subunits was confirmed by modifying the endogenous genes to encode epitope-tagged variants. Open reading frames were tagged at the COOH-terminus with three HA or nine myc epitopes as described above, using a *TRP1* integrative vector. At 37°C, *APC11-myc9* strains arrested in mitosis whereas all other strains grew normally indicating that these tagged proteins were fully functional.

Immunoprecipitates from *APC2-myc9, APC4-myc9, APC5-myc9* and *APC11-myc9* cells (Fig. 9A,B,C) all contained the same set of proteins, which included all known constituents of the yeast APC. The identity of p40 was confirmed by showing that co-precipitation of p40 with Cdc16-myc6p or Apc5-myc9p was dependent on the *APC9* gene (Fig. 9A).

As a first step towards analysing the function of these genes, one copy of each open reading frame in diploid strains was replaced by a *HIS* marker which complements the *his3* mutation. For gene disruption, a cassette containing the coding sequence of the *Schizosaccharomyces pombe his5*^{*+*} gene was amplified by polymerase chain reaction (PCR) from pFA6a-HIS3MX6 with target gene-specific primers and transformed into a diploid strain.

Tetrad analysis revealed that *APC2, APC4, APC5,* and *APC11* are essential genes. His⁺ spores arrested as large, budded cells after 1 to 3 cell divisions after germination.

Haploid cells containing a deletion of *APC9* were viable at 25 and 37°C. It was found, however, that Cdc27p was largely absent in precipitates from-*apc9* strains (Fig. 9A). Apc9p might therefore be required to stabilise the interaction of Cdc27p with the APC. To analyse the function of Apc9p in vivo, small unbudded cells from a *apc9* strain were isolated and their progression through the cell cycle upon incubation at 37°C was followed. *apc9* cells showed a defect in the onset of anaphase as indicated by the accumulation of cells with short spindles and a delay by approximately 15 min of spindle elongation and sister chromatid separation. Exit from mitosis and cytokinesis seemed to occur normally. Although not an essential gene, *APC9* is clearly required for efficient entry into anaphase. Apc9p is not the only non-essential APC subunit. *CDC26* encodes a small, heat-inducible subunit which is only essential at increased temperatures. In immunoprecipitates from *Δcdc26* strains, the amounts of Cdc16p, Cdc27p and Apc9p were severely reduced whereas the other subunits were still found to be associated with each other (Fig. 9C).

Fig. 9 shows the characterisation of the identified proteins as APC subunits. The genes encoding putative APC subunits were replaced by myc-tagged variants. Open reading frames were tagged at the COOH-terminus with three HA or nine myc epitopes as described above, using a *TRP1* integrative vector. At 37°C, *APC11-myc9* strains arrested in mitosis whereas all other strains grew normally indicating that these tagged proteins were fully functional. Cells were labeled with ³⁵S amino acids and processed for immunoprecipitations with an antibody (9E10) to the myc tag. Extracts from wild type (no tag) and *CSE1-myc9* cells served as controls. Proteins whose precipitation is not myc-dependent are marked (*). (A) Co-precipitation of APC subunits with Apc5-myc9p and subunit composition of the APC isolated from *Δapc9* strains. Cells were labeled at 30°C. (B) Identification of Apc11p as an APC subunit. Cells were labeled at 23°C. (C) Co-precipitation of APC subunits with Apc2-myc9p and Apc4-myc9p and subunit composition of the APC isolated from *Δcdc26* strains. Cells were labeled for 90 min at 25°C and then for 60 min at 37°C.

### Example 11

### Characterisation of Apc11p

Databases at the National Center for Biotechnology Information were searched using gapped BLAST (Altschul et al., 1997). ESTs were assembled into contigs using the program AssemblyLIGN (Oxford Molecular Group). ESTs encoding Apc11p homologues: Human, six ESTs including gb AA541685; rat, gb H32307; fruit fly, seven ESTs including gb AA202488; fission yeast, dbj AB001022 (a putative intron was removed). The COOH-terminal 339 aa of the mouse Apc2p homologue are encoded by a contig of 16 ESTs. The cullin homology domain is encoded in gb AA125506. The program CLUSTAL W (Thompson et al, 1994) was used for multiple sequence alignments.

The database searches identified Apc11p as a member of a highly conserved family of proteins containing a RING-H2 domain, a variant of the RING finger (Fig. 10A). Tagging of *APC11* with myc9 (but not with the shorter HA3) caused a defect in the onset of anaphase and exit from mitosis. Incubation at 37°C led to the accumulation of cells with short and long spindles. Extracts from G1 arrested *APC11-myc9* cells shifted to 37°C were defective in the ubiquitination of mitotic cyclins (Fig. 10B).

Fig. 10 shows the characterisation of Apc11p as follows: (A) Homology of Apc11p to a family of RING finger proteins. The 100 NH₂-terminal aa of Apc11p from budding yeast (Sc; total length, 165 aa) were aligned with predicted open reading frames encoded by ESTs from humans (Hs, 84 aa), rat (Rn, 84 aa), fruit fly (Dm, 85 aa), and fission yeast (Sp, 95 aa. The putative zinc binding amino acids conserved in RING finger proteins (Saurin et al., 1996; Borden et al., 1996) are marked with asterisks. Amino acids identical in at least three sequences are boxed. (B) Defective cyclin ubiquitination in extracts from *APC11-myc9* cells. Wild type (*APC11*) and *APC11-myc9* strains of the genotype *MATa Δpep4 Δbar1* were arrested in G₁ with α-factor at 23°C and shifted to 37°C for 30 min. Extracts were incubated with ATP and the indicated HA3-tagged cyclin substrate (Zachariae and Nasmyth, 1996). Cyclin-ubiquitin conjugates were detected by immunoblotting with an antibody to HA. The Clb2ΔDB protein lacks the destruction box.

### Example 12

### Analysis of the sequence and function of Apc2p

Database searches were carried out as in Example 11 and revealed homology of the COOH-terminal region of Apc2p to a putative open reading frame from C. *elegans* (KO6H7.5) and to a mouse protein whose C-terminal 339 amino acids could be assembled from Expressed Sequence Tags (ESTs) (Fig. 11A). The mouse sequence is 96% identical to that of the human Apc2 protein.

To analyse the function of Apc2p, the gene was mutagenised in vitro and two alleles were obtained which conferred cell cycle arrest at 37°C. These alleles were integrated at the *his3* locus of a haploid strain containing a deletion of the genomic *APC2* gene. *APC2* was mutagenized as described (Muhlrad et al., 1992). To construct a strain for plasmid shuffling, the 2.5 kb XhoI to NdeI fragment of *APC2* was replaced by *TRP1* in a strain that is kept alive by a *CEN-URA3* plasmid containing *APC2* on a 4.1 kb ClaI to BglII fragment. A gapped plasmid was prepared by cutting a *CEN-LEU2* plasmid containing the ClaI to BglII *APC2* fragment with XhoI and NdeI. *APC2* sequences from -29 to +2554 (ATG = +1) were amplified by PCR under mutagenic conditions (MacKelvie et al., 1995). PCR product and gapped plasmid were mixed and transformed into the shuffle strain. Selection for resistance to 5-fluoroorotic acid (FOA) was used to evict the *CEN-URA3-APC2* plasmid (Sikorski and Boeke, 1991). Plasmids conferring cell cycle arrest at 37°C were recovered. The wild type gene and the mutant alleles *apc2-1* and *apc2-2* were cloned into a *HIS3* integrative vector and integrated at the *his3* locus of the shuffle strain. Transformants were cured of the *CEN-URA3-APC2* plasmid using FOA.

Sister chromatid separation was monitored by using cells expressing a *tet* repressor-green fluorescent protein (GFP) fusion which binds to an array of 224 *tet* operator sites integrated into the *ura3* locus on chromosome V. At the onset of anaphase in wild type cells, a single 'GFP dot' divides into two dots which move to opposite poles of the spindle. Small, unbudded G1 cells from wild type and *apc2-1* mutant strains grown at 21°C were isolated and their progression through the cell cycle upon incubation at 37°C was followed. Strains were grown in YEP medium (1 % yeast extract, 1 % peptone) containing 2 % raffinose at 21°C. Small, unbudded cells were isolated by centrifugal elutriation (Schwob and Nasmyth, 1993) and inoculated into YEP medium with 2 % glucose at 37°C. A FACScan (Becton-Dickinson) was used for flow cytometric DNA quantitation (Epstein and Cross, 1992). Pds1-myc18p and spindles were detected by indirect immunofluorescence (Zachariae et al., 1996). tetR-GFP signals were observed in ethanol-fixed cells.

In *apc2-1* cells, DNA replication and the formation of mitotic spindles occurred at the same time (relative to budding) as in wild type cells. However, most of the mutant cells failed both to separate sister chromatids and to elongate their spindles. Cytokinesis and re-replication were completely blocked (Fig. 11B,C). Degradation of Pds1p has been shown to be required for entry into anaphase (Cohen-Fix et al., 1996). In wild type cells, Pdslp degradation starts shortly before the onset of anaphase and is completed in cells that have undergone sister chromatid separation (Cohen-Fix et al., 1996). Detection of Pds1-myc18p by immunofluorescence revealed that most *apc2-1* cells arrested with high levels of Pds1p (Fig. 11C). Deletion of the *PDS1* gene allowed *apc2-1* cells to separate sister chromatids (Fig. 11D). However, spindle elongation was slower in *apc2-1 Δpds1* cells than in wild type cells. These data suggest that the inability of *apc2-1* cells to enter anaphase stems primarily from a defect in the degradation of Pds1p. Western blotting showed that *apc2-1* cells are also defective in the degradation of the mitotic cyclin Clb2p and another APC substrate, Ase1p (Juang et al., 1996), whose degradation is important for spindle disassembly. Extracts prepared from G1 arrested cells of *Δapc2 apc2-1* and *Δapc2 apc2-2* strains were defective in the ubiquitination of Clb2p and Clb3p (Fig. 11E). These data suggest that the defect in cyclin proteolysis is due to defective ubiquitination.

Fig. 11 shows the analysis of the sequence and function of Apc2p. (A) A domain with homology to cullins in Apc2p. The sequence of the cullin homology domain of Apc2p was aligned with Apc2p-related sequences from *Caenorhabditis elegans* (CeKO6H7.5) and mouse (MmApc2) and with cullins from various organisms (Altschul et al., 1997; Thompson et al., 1994). Amino acids identical in at least four sequences are boxed. (B) Distribution of the cellular DNA content in *apc2-1* cells at the restrictive temperature. Small, unbudded G₁ cells grown at 21 °C were isolated by centrifugal elutriation and released into fresh medium at 37°C. Strains were grown in YEP medium (1 % yeast extract, 1 % peptone) containing 2 % raffinose at 21°C. Small, unbudded cells were isolated by centrifugal elutriation (Schwob and Nasmyth, 1993) and inoculated into YEP medium with 2 % glucose at 37°C. A FACScan (Becton-Dickinson) was used for flow cytometric DNA quantitation (Epstein and Cross, 1992). Pds1-myc18p and spindles were detected by indirect immunofluorescence. tetR-GFP signals were observed in ethanol-fixed cells. Similar results were obtained with the other *apc2-1* strains. (C) Percentage of cells with buds (rectangles), short spindles (open circles), long spindles (filled circles), separated sister chromatids (two GFP dots, diamonds) after release of small G₁ cells at 37°C of a *apc2-1* strain containing the tetO tetR-GFP system. Cells with staining from Pds1-myc18p (filled triangles) were detected after release of an *apc2-1 PDS1-myc18* strain. Budding and the formation of short and long spindles was almost identical in both strains. (D) Release of small G₁ cells of an *apc2-1 Δpds1* strain containing the tetO tetR-GFP system. (E) Ubiquitination of mitotic cyclins in extracts from wild type (*APC2*), *Δapc2 APC2*, *Δapc2 apc2-1* and *Δapc2 apc2-2* cells. Strains (*MATa Δpep4 Δbar1*) were arrested in G₁ with α-factor at 23°C and shifted to 37°C for 30 min. The ubiquitination assay was carried out as described in the legend to Fig. 10B.

### REFERENCES

Akari H., Awane K., Ogawa N., Oshima Y., *Mol*. *Gen. Genet.* **231,** 329 (1992)
Altschul S. F. et al., *Nucl. Acids Res*. **25,** 3389 (1997)
Amon A., Irniger S., Nasmyth K., Cell **77,** 1037 (1994)
Ausubel F. M., et al., *Current Prot in Mol Biol* 1, (1994)
Bai C. et al., *Cell* **86,** 263 (1996)
Borden K. L. B. and Freemont P. S., *Curr. Opin. Struct. Biol*. **6**, 395 (1996)
Ciechanover A., *Cell* **79,** 13 (1994); J. M. Peters, *Trends Biochem. Sci*. **19,** 377 (1994)
Cohen-Fix O., Peters J.-M., Kirschner M. W., Koshland D., *Genes Dev.* **24,** 3081 (1996)
Cvrckova F. and Nasmyth K., *EMBO J*. **12,** 5277-5286 (1993)
Dahman C., Diffley J. F. X., Nasmyth K., *Curr. Biol*. **5,** 1257 (1995)
Ellison, J. M. and Hochstrasser, M., *Journ Biol Chem* **266,** 21150-21157 (1991)
Engle D. B. *et al*., *J. Biol*. *Chem.* **265,** 16132 (1990)
Epstein C. B. and Cross F. R., *Genes Dev.* **6,** 1695 (1992)
Funabiki H. *et al*., *Nature* **381,** 438 (1996)
Gietz and Sugino, *Gene* **74,** 527 (1988)
Glotzer, M, et al., *Nature* **349,** 132-138 (1991)
Guthrie, C. and Fink, G. R. Guide to Yeast Genetics and Molecular Biology, *Meth. Enzym*. **194** (1991)
Harlow, E. and Lane, D., Antibodies, A Laboratory Manual, Cold Spring Harbor Laboratory (1988)
Hirano T., Hiraoka Y., Yanagida M., *J. Cell Biol.* **106,** 1171 (1988)
Holloway S. L., Glotzer M., King R. W., Murray A. W., *Cell* **73,** 1393 (1993)
Irniger S., S Piatti, C. Michaelis, K. Nasmyth, *Cell* **81,** 269 (1995)
Jackson P. K., *Curr. Biol*. **6,** 1209 (1996)
James S. W., Mirabito **P. M.**, Scacheri P. C., Morris N. R., *J. Cell Sci*. **108,** 3485 (1995)
Juang Y.-L. et al., *Science* **275,** 1311 (1996)
King R. W. *et al., Cell* **81,** 279 (1995)
Kipreos E. T. et al., *Cell* **85,** 829 (1996)
Lamb J. R., Michaud W. A., Sikorski R. S., Hieter P. A., *EMBO J.* **13,** 4321 (1994)
Lamond A. I. and Mann M., *Trends Cell Biol.* **7,** 139 (1997)
Längle-Rouault and Jacobs, *NAR* **23,** 3079-3081 (1995)
Luca F. C., Shibuya E. K., Dohrman C. E., Ruderman J. V., *EMBO J.* **10,** 4311 (1991)
MacKelvie S. H., Andrews P. D., Stark M. J., *Mol. Cell. Biol*. **15,** 3777 (1995)
Mann M. and Wilm M., *Anal. Chem*. **66,** 4390 (1996)
Mathias N. et al., *Mol. Cell. Biol*. **16,** 6634 (1996)
Muhlrad D., Hunter R., Parker R., *Yeast* **8,** 79 (1992)
Murray A. W., M. J. Solomon, M. W. Kirschner, *Nature* **339,** 503 (1989)
Nasmyth K., Adolf G., Lydall D., Seddon A.,- *Cell* **62,** 631 (1990)
Neubauer G. et al., *Proc. Natl. Acad. Sci. U.S.A*. **94,** 385 (1997)
O'Donnell K. L. , et al., *J. Cell Sci*. **99,** 711 (1991)
O'Reilly et al., Baculovirus Expression Vectors. A Laboratory Manual. Ed. Freeman & Co, New York, (1992)
Osmani S. A., Engle D. B., Doonan J. H., Morris N. R., *Cell* **52,** 241 (1988)
Peters J.M., *Trends Biochem. Sci*. **19,** 377 (1994)
Peters J.-M., King R. W., Höög C., Kirschner M. W., *Science* **274,** 1199 (1996)
Rolfe, et al., *PNAS* **92,** 3264-3268 (1995)
Samejima I. and Yanagida M., *J. Cell Biol*. **127,** 1655 (1994)
Saurin A. J., Borden K. L. B., Boddy M. N., Freemont P. *S., TIBS* **21,** 208 (1996)
Schwob E. and Nasmyth K., *Genes Dev.* **7**, 1160 (1993)
Schwob E., Böhm T., Mendenhall M. D., Nasmyth K., *Cell* **79,** 233 (1994)
Sherman F., *Methods Enzymol*. **194,** 3 (1991)
Shevchenko A. et al., *Proc. Natl. Acad. Sci. U.S.A*. **93,** 14440 (1996) a)
Shevchenko A., Wilm M., Vorm O., Mann M., *Anal. Chem.* **68,** 850 (1996) B)
Sikorski and Hieter, *Genetics* **122,** 19-27 (1989)
Sikorski R. S. and Boeke J. D., *Methods Enzymol*. **194,** 302 (1991)
Starborg M., Brundell E., Gell K., Höög C., *J. Biol. Chem.* **269,** 24133 (1994)
Sudakin V. *et al*., *Mol*. *Biol*. *Cell* **6,** 185 (1995)
Surana U., *et al., EMBO J.* **12,** 1969 (1993)
Thompson J. D., Higgins D. G., Gibson T. J., *Nucl*. *Acids Res.* **22,** 4673 (1994)
Tugendreich et al., *PNAS* **90,** 10031-10035 (1993)
Tugendreich S., Tomkiel J., Earnshaw W., Hieter P., *Cell* **81,** 261 (1995)
Wilm M. et al., *Nature* **379,** 466 (1996) a)
Wilm M., Neubauer G., Mann M., *Anal. Chem.* **68,** 527 (1996) b)
Willems et al., *Cell* **86,** 453 (1996)
Zachariae W. and Nasmyth K., *Mol. Biol. Cell* **7,** 791 (1996)
Zachariae W. et al., *Science* **274,** 1201 (1996)

## Claims

1. A method for identifying substances that inhibit rapidly proliferating cells by interfering with the cells' entry into the subsequent cell cycle, **characterized by** the following steps
a) identifying novel subunits of the Anaphase Promoting Complex (APC) by
i) replacing in a cell selected from cells from the budding yeast *Saccharomyces cerevisiae* or from human cells one or more endogenous genes encoding a known APC subunit by epitope-tagged versions of said genes or transforming the cell with a vector containing the corresponding epitope-tagged cDNA(s),
ii) growing cells obtained in i) and preparing a protein extract,
iii) isolating the APC by contacting the protein extract obtained in ii) with an antibody directed against the epitope-tag,
iv) isolating the antibody-bound protein(s) and purifying them,
v) determining the sequence of the protein(s), and optionally
vi) identifying, in the case that the proteins obtained are yeast proteins, the human subunit(s) by comparing the sequence(s) of the yeast protein(s) and/or the DNA sequence encoding those proteins with published human sequences;
b) producing recombinant human or *Saccharomyces cerevisiae* APC containing APC subunits identified in a) by
i) expressing cDNAs encoding APC subunits
ii) isolating and purifying the subunits and allowing them to assemble to form a functional APC;
c) incubating the recombinant APC obtained in b), in the presence of a ubiquitin activating enzyme (E1), a suitable ubiquitin conjugating enzyme (E2), ubiquitin, ATP and an APC substrate, with a test substance, and
d) determining the effect of the test substance on the APC's ability to ubiquitinate the substrate.

2. The method of claim 1, wherein the cell of step a i) is a yeast cell and the gene(s) are selected from the group *APC1, CDC16, CDC23, CDC26, CDC27, APC2, APC5*, *APC4, APC9 and APC11.*

3. The method of claim 1, wherein the cell of step a i) is a human cell and the gene(s) or the respective cDNAs are selected from a group of genes that are homologs of the yeast genes *APC1, CDC16, CDC23, CDC26, CDC27, APC2, APC5, APC4, APC9 and APC11.*

4. The method of claim 1, wherein the cDNAs in step b i) are expressed in a Baculovirus expression system.

5. The method of claim 1, wherein the APC of step b) and c) and the substrate of step c) are of human origin.

6. The method of claim 1 or 5, wherein said APC substrate is cyclin B.

7. The method of claim 6, wherein the substrate is a recombinant cyclinB.

## Patentansprüche

1. Verfahren zur Identifizierung von Substanzen, die rasch proliferierende Zellen hemmen, indem sie den Eintritt der Zellen in den anschließenden Zellzyklus stören, **gekennzeichnet durch** die folgenden Stufen:
a) Identifizierung neuer Untereinheiten des Anaphase-einleitenden Komplexes (APC) **durch**
i) Ersetzen in einer Zelle, die unter Zellen der knospenden Hefe *Saccharomyces cerevisiae* oder unter humanen Zellen ausgewählt ist, eines oder mehrerer endogener Gene, die eine bekannte APC-Untereinheit kodieren, **durch** Epitop-markierte Versionen der Gene oder Transformation der Zelle mit einem Vektor, der die entsprechende(n) Epitop-markierte(n) cDNA(s) enthält;
ii) Züchtung der unter i) erhaltenen Zellen und Herstellung eines Proteinextrakts;
iii) Isolierung des APC **durch** Kontaktieren des Proteinextrakts, der unter ii) erhalten wird, mit einem Antikörper, der gegen die Epitop-Markierung gerichtet ist;
iv) Isolierung des/der Antikörper-gebundenen Proteins/Proteine und deren Reinigung;
v) Bestimmung der Sequenz des Proteins/der Proteine; und gegebenenfalls
vi) Identifizierung der humanen Untereinheit(en) für den Fall, dass die erhaltenen Proteine Hefeproteine sind, **durch** Vergleich der Sequenz(en) des Hefeprotein/der Hefeproteine und/oder der DNA-Sequenz, die diese Proteine kodiert, mit veröffentlichten Humansequenzen;
b) Herstellung von rekombinantem humanem oder *Saccharomyces cerevisiae*-APC, enthaltend APC-Untereinheiten, die unter a) identifiziert wurden, **durch**
i) Expression von cDNAs, die APC-Untereinheiten kodieren;
ii) Isolierung und Reinigung der Untereinheiten und Ermöglichung von deren Zusammensetzung unter Bildung eines funktionellen APC;
c) Inkubation des unter b) erhaltenen rekombinanten APC in Gegenwart eines Ubiquitin aktivierenden Enzyms (E1), eines geeigneten Ubiquitin konjugierenden Enzyms (E2), von Ubiquitin, ATP und einem APC-Substrat mit einer Testsubstanz und
d) Bestimmung der Wirkung der Testsubstanz auf die Fähigkeit des APC, das Substrat zu ubiquitinieren.

2. Verfahren nach Anspruch 1, wobei es sich bei der Zelle von Stufe a i) um eine Hefezelle handelt und das/die Gene aus der Gruppe APC1, CDC16, CDC23, CDC26, CDC27, APC2, APC5, APC4, APC9 und APC11 ausgewählt sind.

3. Verfahren nach Anspruch 1, wobei es sich bei der Zelle von Stufe a i) um eine humane Zelle handelt und das Gen/die Gene oder die entsprechenden cDNAs aus der Gruppe der Gene ausgewählt sind, die Homologe der Hefegene APC1, CDC16, CDC23, CDC26, CDC27, APC2, APC5, APC4, APC9 und APC11 sind.

4. Verfahren nach Anspruch 1, wobei die cDNAs in Stufe b i) in einem Baculovirus-Expressionssystem exprimiert werden.

5. Verfahren nach Anspruch 1, wobei der APC von Stufe b) und c) und das Substrat von Stufe c) humanen Ursprungs sind.

6. Verfahren nach Anspruch 1 oder 5, wobei es sich bei dem APC-Substrat um Cyclin B handelt.

7. Verfahren nach Anspruch 6, wobei es sich bei dem Substrat um ein rekombinantes Cyclin B handelt.

## Revendications

1. Procédé pour identifier des substances qui inhibent les cellules proliférant rapidement en interférant avec les entrées des cellules dans le cycle cellulaire ultérieur, **caractérisé par** les étapes suivantes :
a) identification de nouvelles sous-unités du complexe de promotion de l'anaphase (APC) par
i) replacement dans une cellule, choisie parmi des cellules de la levure bourgeonnée Saccaromyces cerevisiae ou des cellules humaines d'un ou de plusieurs gènes codant pour une sous-unité connue de l'APC, par des versions marquées par un épitope desdits gènes ou transformant la cellule par un vecteur contenant le ou les cADN marqués par un épitope correspondant,
ii) culture des cellules obtenues sous i) et préparation d'un extrait protéinique
iii) isolement de l'APC en mettant en contact l'extrait protéinique obtenu dans ii) avec un anticorps dirigé contre l'épitope marqueur
iv) isolement de la ou des protéines liées sur l'anticorps et purification de celle(s)-ci,
v) détermination de la séquence de la ou des protéines et éventuellement
vi) identification, dans le cas où les protéines obtenues seraient des protéines de levure, de la ou des sous-unités humaines en comparant la ou les séquences de la ou des protéines de levure et/ou la séquence d'ADN codant pour ces protéines avec des séquences humaines publiées ;
b) production de l'APC recombinant humain ou de Saccaromyces cerevisiae contenant des sous-unités de l'APC identifiées sous a) par
i) l'expression des cADN codant pour des sous-unités de l'APC,
ii) l'isolement et la purification des sous-unités et en permettant leur assemblage pour former un APC fonctionnel ;
c) incubation de l'APC recombinant obtenu sous b), en présence d'une enzyme activant l'ubiquitine (E1), d'une enzyme appropriée conjuguant l'ubiquitine (E2), d'ubiquitine, d'ATP et d'un substrat d'APC avec une substance test et
d) détermination de l'effet de la substance test sur l'aptitude de l'APC à ubiquitiner le substrat.

2. Procédé de la revendication 1, dans lequel la cellule de l'étape a i) est une cellule de levure et le ou les gènes sont sélectionnés parmi le groupe APC1, CDC16, CDC23, CDC26, CDC27, APC2, APC5, ACP4, APC9 et APC11.

3. Procédé de la revendication 1, dans lequel la cellule de l'étape a i) est une cellule humaine et le ou les gènes ou les cADN respectifs sont choisis parmi un groupe de gènes qui sont des homologues des gènes de levure APC1, CDC16, CDC23, CDC26, CDC27, APC2, APC5, ACP4, APC9 et APC11.

4. Procédé de la revendication 1, dans lequel les cADN dans l'étape b i) sont exprimés dans un système d'expression Baculovirus.

5. Procédé de la revendication 1, dans lequel les cADN des étapes b) et c) sont d'origine humaine.

6. Procédé de la revendication 1 ou 5, dans lequel ledit substrat d'APC est la cycline B.

7. Procédé de la revendication 6, dans lequel le substrat est une cycline B recombinante.
